# EUROPEAN PATENT APPLICATION

(11) **EP 3 567 059 A1**
(43) Date of publication of application: **13.11.2019**
(21) Application number: 18305575.5
(22) Date of filing: 09.05.2018
(51) Int. Cl.: C08B 37/16

(54) **SUBSTITUTED CYCLODEXTRIN-METAL COMPLEXES AND USES THEREOF**

(71) Applicant: Biocydex, 86000 Poitiers (FR)
(72) Inventor: BELGSIR, El Mustapha, 86000 Poitiers (FR); TURPIN, Frédéric, 37000 Tours (FR)
(74) Representative: Icosa

(57) **Abstract**

The present invention relates to a complex of Ag, Zn, Cu, Pt, Au, oxides, hydroxides and salts thereof; with a cyclodextrin of formula (I) as defined in claim 1. Advantageously, the complex further comprises an organic therapeutic agent, preferably an antibiotic agent. The complexation according to the invention advantageously maintains the metal: organic therapeutic agent molar ratio. Even more advantageously, the complexation according to the invention enhances the intrinsic aqueous solubility of the metal-organic therapeutic agent association. The present invention further relates to compositions, pharmaceutical compositions and adequate formulations and uses thereof as well as devices comprising a complex of the invention. Lastly, the invention relates to a method for improving the aqueous solubility of a metal selected from Ag, Zn, Cu, Pt, Au, Ru, As, Sb, Bi, Ti, V, Ni, Hg, Pb, Co, oxides, hydroxides and salts thereof by providing a reaction mixture comprising said metal, an aqueous medium and a cyclodextrin of formula (I) as defined in claim 1.

## Description

### FIELD OF INVENTION

The present invention relates to complexes of substituted cyclodextrins with metals. The complexes of the present invention are of particular interest as they enhance the aqueous solubility of metals or metal-associated organic compounds. Compositions comprising the substituted cyclodextrin complexes according to the present invention open up new perspectives for the application of metal and metal-associated organic compounds whose use is restrained due to their limited concentration in aqueous media, particularly in the field of therapeutic applications.

### BACKGROUND OF INVENTION

Inorganic medicinal chemistry is considered as a promising discipline for enriching the therapeutic armamentarium against a broad range of pathologies ever since the ancient civilizations of Mesopotamia, Egypt, India and China up to Elrich's first arsenic-based treatment against syphilis.

The field of inorganic medicinal chemistry in medicine may usefully be divided into two main categories. On the one hand, metal ions, oxides or salts thereof to be used as such and on the other hand, metal-based drugs and imaging agents where the central metal ion is one of the pharmacological key-features of the drug.

Nowadays, the specific interaction between metals and biomolecules render inorganic medicinal chemistry a promising field for the new drug discovery and the optimization of existing treatments. Current therapeutic strategies involve metal agents in the treatment of bacterial, parasitic and fungal infections, as well as in the treatment of cancer or even diabetes, to name but a few.

Nevertheless, the development of metals or metal-complexes as drugs is a challenging task, namely because of the poor solubility of the metallic therapeutic agents within the aqueous media, such as the biological systems.

As in many solubility enhancement approach strategies, vectorization of the metal-comprising-therapeutic agent seems a promising getaway solution to overcome solubility issues. Such strategies comprise the cyclodextrin vectorization of metallic agents or the use of biphasic formulations such as suspensions or emulsions.

International patent application WO2004071639 discloses biopolymer functionalized (3,6-anhydro)-cyclodextrins for the decontamination of the human body from metals, namely radioactive metals. However, this reference does not teach how to enhance the solubility of an inorganic agent to be administered to the human body, released therein and exerting its pharmacological effects. As generally accepted in the art, a non-reversible and voluminous vectorization hinders the metal agent from reaching its biological target.

Surprisingly, the present invention supplies reversible cyclodextrin-metal complexes that are biocompatible, non-toxic and therapeutically effective.

As previously discussed, current therapeutics use metal-based drugs in a broad spectrum of treatments. For example, silver sulfadiazine is a topical antibiotic used in partial thickness and full thickness burns to prevent infection. Silver sulfadiazine, initially discovered in the 1960s, is currently on the World Health Organization's List of Essential Medicines, considered among the most effective and safe medicines needed in a health system.

Biphasic pharmaceutical formulations cannot be a universal solution to a therapeutic agent's aqueous solubility problems. One the one hand, suspensions and emulsions are restrained to a limited number of administration routes, excluding for example intravenous administration or local application to sensitive tissues. On the other hand, the dispersion of the metallic agent in the non-continuous phase of the biphasic system reduces the bioavailability of the agent. Up to the present invention, the aqueous solubility of silver sulfadiazine was overcome by its formulation into lipid emulsions (such as polysorbate, cetyl alcohol or paraffine based creams) commercialized as Flammazine®, Flamazine® or Silvadene®. In the latter, silver sulfadiazine is dispersed, predominantly in the form of microparticles. However, such formulations not only limit the administration route options and the applicable administration concentration ranges, but also trigger adverse effects to the vulnerable burnt skin. In fact, silver sulfadiazine particles possess abrasive properties, and lipophilic excipients of silver sulfadiazine creams tend to accumulate on the affected skin, further causing irritation and possibly infections of the burn wounds if not thoroughly rinsed.

Advantageously, the cyclodextrin-metal complexes of the present invention can play a dual role, vectorizing at least one metal associated to an organic therapeutic agent. Thus, the Applicant has developed a functionalized-cyclodextrin vector that can solubilize both metal and metal-based drugs. Even more advantageously, the cyclodextrin-metal complexes of the present invention can be formulated into aqueous formulations such as skin, ocular or auricular solutions. Such complexes, give way to further therapeutic applications of metals associated to an organic therapeutic agent whose potential was limited due to their limited aqueous solubility.

Hegazy *et al.* discloses cyclodextrin-silver sulfadiazine complexes with the aim of enhancing the solubility of silver sulfadiazine. The latter complexes consist of natural beta cyclodextrin complexes, as well as functionalized beta cyclodextrins such as hydroxypropyl-beta-cyclodextrins and carboxymethyl-beta-cyclodextrins (Hegazy et al., 2013. Int J Pharm Pharm Sci. 5(1):461-468). However, the aforementioned complexes do not go beyond the intrinsic solubility of silver sulfadiazine. Furthermore, the simultaneous complexation of both silver and sulfadiazine at the molar ratio of 1:1 is not provided.

Surprisingly, the complexes of the present invention boost the aqueous solubility of silver sulfadiazine by a factor of more than 5x10⁴. Even more surprisingly, the Applicant has demonstrated that the complexes of the present invention maintain the initial ratio between the metal and the organic agent past the preparation of the cyclodextrin-metal-organic agent complexes. This advantageous property of the invention is of particular interest in terms of therapeutic efficacy and stability of the formulations comprising the complexes of the invention.

### SUMMARY

The present invention relates to a complex of a metal selected from the group comprising Ag, Zn, Cu, Pt, Au, oxides, hydroxides and salts thereof; with a cyclodextrin of formula (I) 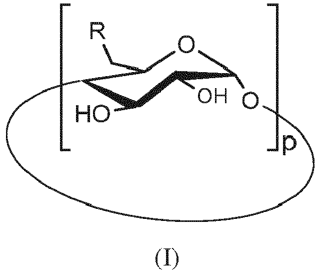 wherein:
p is 6, 7 or 8;
R is -OH or -X-NH-Z-NHR₁;
X is a single bond or R₂;
Z is selected from -C=O and -C=S;
R₁ is selected from H and optionally substituted short chain alkyls; preferably
R₁ is selected from methyl, ethyl propyl and butyl;
R₂ is selected from optionally substituted short chain alkyls; preferably R₂ is selected from methyl, ethyl and propyl;
with the proviso that at least one R = -X-NH-Z-NHR₁.

In one embodiment,
p is 7;
R is -OH or -NH-Z-NHR₁;
Z is -C=S;
R₁ is methyl;
with the proviso that at least one cyclodextrin monomer has R = -NH-Z-NHR₁.

In one embodiment, the cyclodextrin of the complex is a beta-cyclodextrin bearing at least one substituent R: -NHC=SNHCH₃.

In one embodiment, the complex of the invention further comprises at least one organic therapeutic agent, said organic therapeutic agent being selected from antibiotic, anti-fungal, antiviral and antiparasitic agents; salts thereof and combinations thereof.

In one embodiment, the complex of the invention comprises:
- a metal being Ag, oxide, hydroxide or salts thereof, and
- an organic therapeutic agent being an antibiotic agent, preferably a sulfamide, even more preferably the organic therapeutic agent is sulfadiazine.

In one embodiment, the complex of the invention comprises:
- a metal being Ag;
- an organic therapeutic agent being a sulfamide, preferably sulfadiazine;
- a cyclodextrin of formula (I), wherein:
   p is 7;
   R is -OH or -NH-Z-NHR₁;
   Z is -C=S; and R₁ is methyl;
   with the proviso that at least one beta-cyclodextrin monomer has R = -NH-Z-NHR₁; and
wherein the molar ratio between the organic therapeutic agent and the metal is ranging from about 200 to about 1, preferably from about 20 to about 2, even more preferably from about 2 to 1.

The present invention also relates to a composition comprising the complex according to the present invention.

In one embodiment, the composition comprises:
- a metal being Ag;
- a cyclodextrin of formula (I), wherein:
   p is 7;
   R is -OH or -NH-Z-NHR₁;
   Z is -C=S;
   R₁ is methyl;
   with the proviso that at least one beta-cyclodextrin monomer has R = -NH-Z-NHR1;
wherein:
- when the complex comprises at least one organic therapeutic agent, said organic therapeutic agent is sulfadiazine; and
- the molar ratio between sulfadiazine and Ag is ranging from about 2 to about 1, preferably from about 1.5 to about 1; and
- the concentration of silver sulfadiazine in the composition is ranging from about 1.0 mg/L to about 40 g/L; preferably ranging from about 1.0 to about 20 g/L.

In one embodiment, the composition further comprises at least one active agent selected from antiseptics, antibiotics, anti-inflammatories, and soothing agents.

The present invention also relates to a pharmaceutical composition comprising the composition according to the present invention, and at least one pharmaceutically acceptable excipient.

In one embodiment, the pharmaceutical composition according to the present invention is in a pharmaceutical form selected from aqueous solutions, sprays, gels, hydrogels, liquid soap formulations, eye drops, ear drops and oil-in-water emulsions.

The present invention also relates to the complex, the composition or the pharmaceutical composition according to the present invention, for use as a drug.

In one embodiment, the complex, the composition or the pharmaceutical composition according to the present invention is/are for use in the prevention and/or the treatment of skin infections.

The present invention also relates to a device comprising the complex, the composition or the pharmaceutical composition according to the present invention; preferably the device is a wound dressing; even more preferably the device is a dermal patch.

The present invention also relates to a method for improving the aqueous solubility of a metal selected from the group comprising Ag, Zn, Cu, Pt, Au, Ru, As, Sb, Bi, Ti, V, Ni, Hg, Pb, Co, oxides, hydroxides and salts thereof;
wherein the method comprises providing a reaction mixture comprising said metal, an aqueous medium and a cyclodextrin of formula (I): 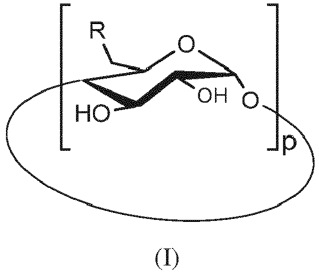 wherein:
p is 5, 6, 7 or 8;
R is -OH or -X-NH-Z-NHR₁
X is a single bond or R₂;
Z is selected -C=O and -C=S;
R₁ is selected from H or optionally substituted short chain alkyls; preferably
R₁ is selected from methyl, ethyl and propyl; and
R₂ is selected optionally substituted short chain alkyls; preferably R₁ is selected from methyl, ethyl and propyl;
with the proviso that at least one beta-cyclodextrin monomer has R: -X-NH-Z-NHR₁.

In one embodiment, the metal is Ag and the reaction mixture further comprises at least one organic therapeutic agent, wherein the organic therapeutic agent is selected from antibiotic, anti-fungal, antiviral and antiparasitic agents; salts thereof and combinations thereof; preferably the organic therapeutic agent is sulfamide, even more preferably the organic therapeutic agent is sulfadiazine.

### DEFINITIONS

In the present invention, the following terms have the following meanings:
- **"About"** is used herein to mean approximately, roughly, around, or in the region of. When the term "about" is used in conjunction with a numerical range, it modifies that range by extending the boundaries above and below the numerical values set forth.
- **"Alkyl"** refers to any saturated linear or branched hydrocarbon chain, with 1 to 12 carbon atoms, preferably 1 to 6 carbon atoms. Short chain alkyl group refer to any saturated linear or branched hydrocarbon chain with 1 to 14 carbon atoms, preferably methyl, ethyl, propyl, isopropyl, *n*-butyl, *sec*-butyl, isobutyl and *tert*-butyl.
- **"Alkylamino"** refers to any N-alkyl group. In embodiments of the invention, a substituted alkyl group is an alkylamino group, preferably a methylamino selected from the group comprising or consisting of methylamino, ethylamino, propylamino, isopropylamino, *n*-butylamino, *sec*-butylamino, isobutylamino and *tert*-butylamino.
- **"Alkyloxy"** refers to any O-alkyl group. In embodiments of the invention, a substituted alkyl group is an alkylamino group, preferably a methylamino selected from the group comprising or consisting of methoxy, ethoxy, propyloxy, isopropyloxy, *n*-butyloxy, *sec*-butyloxy, isobutyloxy and *tert*-butyloxy.
- **"Aqueous medium"** refers to a medium essentially comprising or consisting of water. An aqueous medium may further comprise sugars, lipids, mineral elements, biopolymers, cells and debris thereof. In one embodiment, the aqueous medium is an aqueous solution. In one embodiment, the aqueous medium is the continuous phase of an oil-in water emulsion. In one embodiment, the aqueous medium is situated in a biological tissue or fluid such as gastrointestinal fluids, blood, blood plasma or wound exudates. In one embodiment, the aqueous medium is situated on the wounded or burnt skin.
- **"Complex"** refers herein to a molecule binding to a metal ion and/or an organic therapeutic agent. Complexation (also termed vectorization) involves the formation or presence of one or more non-covalent bonds between the complexation agent (vector) with a metal ion and/or an organic therapeutic agent. In one embodiment, the vector is a cyclodextrin. In one embodiment, the complex is a metal-cyclodextrin complex. In one embodiment, the complex is a metal-organic therapeutic agent-cyclodextrin complex.
- **"Cyclodextrin"** refers to a compound made up of sugar molecules bound together in a ring. Cyclodextrins are composed of 6 or more α-D-glucopyranoside units linked by α-1→4 glucosidic bonds. Typical cyclodextrins contain a number of glucose monomers ranging from six to eight units in a ring, creating a cone shaped polymer. Alpha-cyclodextrins (α-cyclodextrins) comprise 6 α-D-glucopyranoside units. Beta-cyclodextrins (β-cyclodextrins) comprise 7 α-D-glucopyranoside units and gamma-cyclodextrins (γ-cyclodextrins) comprise 8 α-D-glucopyranoside units. In one embodiment, the cyclodextrin according to the invention is a chemically modified cyclodextrin.
- "**Intrinsic solubility**" is the equilibrium solubility of the free acid or free base form of an ionizable compound at a pH where it is fully un-ionized.
- **"Pharmaceutically acceptable excipient"** refers to an excipient that does not produce an adverse, allergic or other untoward reaction when administered to an animal, preferably a human. It includes any and all solvents, dispersion media, coatings, antibacterial and antifungal agents, isotonic and absorption delaying agents and the like. For human administration, preparations should meet sterility, pyrogenicity, general safety and purity standards as required by FDA Office of Biologics standards.
- "**Subject**" refers to an animal, including a human. In the sense of the present invention, a subject may be a patient, *i.e.,* a person receiving medical attention, undergoing or having underwent a medical treatment, or monitored for the development of a disease. In one embodiment, the subject is a burnt-skin patient.
- "**Therapeutic agent**", as used herein, describes a molecule or a substance, preferably a chemical entity such as a metal, an organic entity ("**Organic therapeutic agent**") or a metal-organic therapeutic agent association, whose administration to a subject slows down or stops the progression, aggravation, or deterioration of one or more symptoms of a disease, or condition; alleviates the symptoms of a disease or condition; cures a disease or condition. In one embodiment, the therapeutic agent is a metal-cyclodextrin complex. In one embodiment, the therapeutic agent is a metal-organic therapeutic agent-cyclodextrin complex.
- **"Therapeutically effective amount"** refers to the amount of a therapeutic agent necessary and sufficient for slowing down or stopping the progression, aggravation, or deterioration of one or more symptoms of the disease, or condition; alleviating the symptoms of the disease or condition; and/or curing the disease or condition.
- **"Topical administration"** characterizes the delivery, administration or application of a composition directly to the site of interest (*e.g.,* the skin) for a localized effect. Preferably, topical administration is carried out without any significant absorption of components of components of the composition into the subject's blood stream (to avoid a systemic effect).
- **"Treatment", "treating", "treatment"** or **"alleviation"** refer to both therapeutic treatment and prophylactic or preventative measures ("**Prevention**"; wherein the object is to prevent or slow down (lessen) the targeted pathologic condition or disorder). Subjects in need of treatment include those already diagnosed with the disorder as well as those prone to have the disorder or those in whom the disorder is to be prevented. In one embodiment, the treatment according to the present invention refers to an infection treatment and/or prevention. In one embodiment, the treatment according to the present invention refers to a burnt-skin infection treatment and/or prevention. A subject is successfully "treated" for an infection or burnt skin if, after receiving a therapeutically effective amount of a complex, composition, pharmaceutical composition or medicament according to the invention, the subject shows observable and/or measurable reduction in or absence of one or more of the following: reduction in the number of pathogenic cells; reduction in the percent of total cells that are pathogenic; decrease average size of burnt surfaces; reduced redness and/or swelling; and/or relief to some extent, one or more of the symptoms associated with the specific disease or condition; reduced morbidity and mortality, and improvement in quality of life issues. The above parameters for assessing successful treatment and improvement in the disease are readily measurable by routine procedures familiar to a physician.

### DETAILED DESCRIPTION

The present invention relates to a complex of a metal, oxides, hydroxides and salts thereof, with a substituted cyclodextrin.

The Applicant has developed cyclodextrin-metal complexes using substituted cyclodextrins capable of solubilizing metals, namely metals of therapeutic interest in aqueous media.

According to a first embodiment, the cyclodextrin is a cyclodextrin of formula (I) 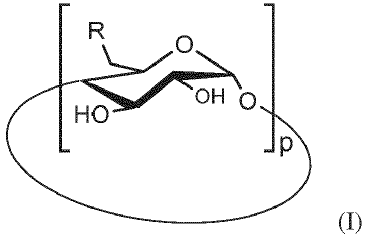 wherein:
p is 6, 7 or 8;
R is -OH or -X-NH-Z-NHR₁;
X is a single bond or R₂;
R₂ is selected from, short chain alkyls optionally substituted; preferably R₂ is selected from methyl, ethyl, propyl and butyl;
Z is selected from -C=O and -C=S;
R₁ is selected from H and optionally substituted short chain alkyls; preferably R₁ is selected from methyl, ethyl propyl and butyl;
with the proviso that at least one R = -X-NH-Z-NHR₁.

In one embodiment, the cyclodextrins of the present invention can be α-cyclodextrins, β-cyclodextrins or γ-cyclodextrins. In such embodiment, p is 6, 7 or 8 respectively. In one particular embodiment, the cyclodextrin is a β-cyclodextrin, wherein p=7.

According to the prior art, natural cyclodextrins are not advantageous in the enhancement of the aqueous solubility of metals. Accordingly, the cyclodextrins of the present invention bear at least one R = -X-NH-Z-NHR₁.

According to a first embodiment, the cyclodextrin is a mono-substituted cyclodextrin bearing one R = -X-NH-Z-NHR₁.

According to a second embodiment, the cyclodextrin is a bi-substituted cyclodextrin bearing two R = -X-NH-Z-NHR₁.

According to a third embodiment, the cyclodextrin is a tri-substituted cyclodextrin bearing three R = -X-NH-Z-NHR₁.

In further embodiments, the cyclodextrin is a pluri-substituted cyclodextrin bearing at least four, five or six R = -X-NH-Z-NHR₁.

In one embodiment where β-cyclodextrins are used, the cyclodextrin of formula (I) bears at least one, two, three, four, five, six or seven R = -X-NH-Z-NHR₁.

In a particular embodiment, the cyclodextrin is a mono-substituted β-cyclodextrin bearing one R = -X-NH-Z-NHR₁. In another particular embodiment, the cyclodextrin is a bi-substituted β-cyclodextrin bearing two R = -X-NH-Z-NHR₁.

In one embodiment, the cyclodextrin substituent R is a thiourea or urea derivative. Accordingly, in one embodiment, Z is from selected from -C=O and -C=S.

According to a first embodiment, Z is -C=O. According to a second embodiment, Z is-C=S.

In one embodiment, the substituent is directly linked to the cyclodextrin. Accordingly, in such embodiment, X represents a single bond.

In one embodiment, the cyclodextrin is a cyclodextrin of formula (I), wherein p = 7, bearing at least one R = -X-NH-Z-NHR₁, wherein X represents a single bond, Z is -C=S, and R1 is methyl.

In one embodiment, the cyclodextrin is a mono-substituted cyclodextrin of formula (I), wherein p = 7, bearing one R = -X-NH-Z-NHR₁, wherein X represents a single bond, Z is -C=S, and R1 is methyl.

In one embodiment, the cyclodextrin is a bis-substituted cyclodextrin of formula (I), wherein p = 7, bearing two R = -X-NH-Z-NHR₁, wherein X represents a single bond, Z is -C=S, and R1 is methyl.

In one embodiment, the cyclodextrin is a tri-substituted cyclodextrin of formula (I), wherein p = 7, bearing three R = -X-NH-Z-NHR₁, wherein X represents a single bond, Z is -C=S, and R1 is methyl.

Alternatively, the substituent can be bound to the cyclodextrin via a short alkyl chain R₂. According to such embodiment, the short alkyl chain R₂ may optionally be substituted by heteroatoms, preferably selected from O, N and S. According to a first embodiment, R₂ is a short alkyl chain selected from the group comprising or consisting of methyl, ethyl propyl and butyl. According to a second embodiment, R₂ is a short alkyl chain selected from the group comprising or consisting of methyl, ethyl and propyl. According to a third embodiment, R₂ is a short alkyl chain selected from the group comprising or consisting of methyl and ethyl. According to a fourth embodiment, R₂ is a methyl group.

According to another embodiment, R₂ is a substituted short alkyloxy chain selected from the group comprising or consisting of methyloxy, ethyloxy, propyloxy and butyloxy. According to a second embodiment, R₂ is a short alkyloxy chain selected from the group comprising or consisting of methyloxy, ethyloxy and propyloxy. According to a third embodiment, R₂ is a short alkyloxy chain selected from the group comprising or consisting of methyloxy and ethyloxy. According to a fourth embodiment, R₂ is a methyloxy group.

According to another embodiment, R₂ is a substituted short alkylamino chain selected from the group comprising or consisting of methylamino, ethylamino, propylamino and butylamino. According to a second embodiment, R₂ is a short alkylamino chain selected from the group comprising or consisting of methylamino, ethylamino and propylamino. According to a third embodiment, R₂ is a short alkylamino chain selected from the group comprising or consisting of methylamino and ethylamino. According to a fourth embodiment, R₂ is a methylamino group.

In one embodiment, the terminal substituent of the thiourea or urea moiety R₁ is selected from the group comprising or consisting of H and optionally substituted short chain alkyls. According to a first embodiment, R₁ is H. According to a second embodiment, R₁ is a short chain alkyl selected from the group comprising or consisting of methyl, ethyl propyl and butyl. According to a third embodiment, R₁ is a short chain alkyl selected from the group comprising or consisting of methyl, ethyl, propyl and butyl. According to a fourth embodiment, R₁ is a short chain alkyl selected from the group comprising or consisting of methyl, ethyl and propyl. According to a fifth embodiment, R₁ is a short chain alkyl selected from the group comprising or consisting of methyl and ethyl. According to a sixth embodiment, R₁ is methyl.

In another embodiment, R₁ is a substituted short alkyloxy chain selected from the group comprising or consisting of methyloxy, ethyloxy, propyloxy and butyloxy. According to a second embodiment, R₁ is a short alkyloxy chain selected from the group comprising or consisting of methyloxy, ethyloxy and propyloxy. According to a third embodiment, R₁ is a short alkyloxy chain selected from the group comprising or consisting of methyloxy and ethyloxy. According to a fourth embodiment, R₁ is a methyloxy group.

In another embodiment, R₁ is a substituted short alkylamino chain selected from the group comprising or consisting of methylamino, ethylamino, propylamino and butylamino. According to a second embodiment, R₁ is a short alkylamino chain selected from the group comprising or consisting of methylamino, ethylamino and propylamino. According to a third embodiment, R₁ is a short alkylamino chain selected from the group comprising or consisting of methylamino and ethylamino. According to a fourth embodiment, R₁ is a methylamino group.

The substituted cyclodextrins according to the present invention can be manufactured by any process known in the art. In one embodiment, the cyclodextrins of the present invention can be manufactured by the process disclosed in US patent US6,080,733. In brief, the substituted cyclodextrin is obtained from the adequate amino-cyclodextrin that is subjected to a reaction with an alkyl-isothiocyanate in an organic solvent such as pyridine at room temperature.

In one embodiment, the metal, oxide, hydroxide or salt thereof is selected from the group comprising or consisting of monovalent, bivalent and trivalent metals, oxides, hydroxides and salts thereof.

Without willing to be bound to a theory, the substituent of the cyclodextrins according to the present invention complexes the metal, oxide, hydroxide or salt thereof thanks to the unbound electrons of the aforementioned "R" substituent moieties.

In one embodiment, the metal, oxide, hydroxide or salt thereof is selected from the group comprising or consisting of monovalent and bivalent metals, oxides, hydroxides and salts thereof. In one embodiment, the metal, oxide, hydroxide or salt thereof is selected from the group comprising or consisting of monovalent metals, oxides, hydroxides and salts thereof. In one embodiment, the metal, oxide, hydroxide or salt thereof is selected from the group comprising or consisting of bivalent metals, oxides, hydroxides and salts thereof.

In a preferred embodiment, the metal, oxide, hydroxide or salt thereof, complexed by the cyclodextrins of the present invention, is selected from the group comprising or consisting of metals, oxides, hydroxides or salts thereof used in inorganic medicinal chemistry. The latter comprise metals, oxides, hydroxides and salts thereof already in use, as well as currently developed metals, oxides, hydroxides and salts thereof for use in therapy.

According to a first embodiment, the metal, oxide, hydroxide or salt thereof is selected from the group comprising or consisting of Ag, Zn, Cu, Pt, Au, Ru, As, Sb, Bi, Ti, V, Ni, Hg, Pb, Co, oxides, hydroxides and salts thereof.

According to a second embodiment, the metal, oxide, hydroxide or salt thereof is selected from the group comprising or consisting of Ag, Zn, Cu, Pt, Au, Ru, As, Sb, Bi, oxides, hydroxides and salts thereof.

According to a third embodiment, the metal, oxide, hydroxide or salt thereof is selected from the group comprising or consisting of Ag, Zn, Cu, Pt, Au, Ru, As, oxides, hydroxides and salts thereof.

According to a fourth embodiment, the metal, oxide, hydroxide or salt thereof is selected from the group comprising or consisting of Ag, Zn, Cu, Pt, Au, oxides, hydroxides and salts thereof.

According to a fifth embodiment, the metal, oxide, hydroxide or salt thereof is selected from the group comprising or consisting of Ag, Zn, Cu, Pt, oxides, hydroxides and salts thereof.

According to a sixth embodiment, the metal, oxide, hydroxide or salt thereof is selected from the group comprising or consisting of Ag, Zn, Cu, oxides, hydroxides and salts thereof.

According to a seventh embodiment, the metal, oxide, hydroxide or salt thereof is selected from the group comprising or consisting of Ag, Zn, Cu, oxides, hydroxides and salts thereof.

In one embodiment, the metal, oxide, hydroxide or salt thereof is selected from the group comprising or consisting of Ag, oxides, hydroxides and salts thereof. In one embodiment, the metal is Ag.

In one embodiment, the metal, oxide, hydroxide or salt thereof is selected from the group comprising or consisting of Zn, oxides, hydroxides and salts thereof. In one embodiment, the metal is Zn.

In one embodiment, the metal, oxide, hydroxide or salt thereof is selected from the group comprising or consisting of Cu, oxides, hydroxides and salts thereof. In one embodiment, the metal is Cu.

In one embodiment, the invention relates to a complex of a metal selected from the group comprising or consisting of Ag, Zn, Cu, Pt, Au, Ru, As, Sb, Bi, Ti, V, Ni, Hg, Pb, Co, oxides, hydroxides and salts thereof; and a cyclodextrin of formula (I) 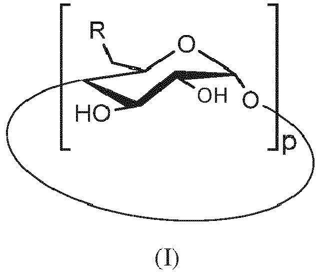 wherein:
p is 6, 7 or 8;
R is -OH or -X-NH-Z-NHR₁;
X is a single bond or R₂;
Z is selected from -C=O and -C=S;
R₁ is selected from H and optionally substituted short chain alkyls; preferably R₁ is selected from methyl, ethyl propyl and butyl;
R₂ is selected from optionally substituted short chain alkyls; preferably R₂ is selected from methyl, ethyl and propyl;
with the proviso that at least one R = -X-NH-Z-NHR₁.

In one embodiment, the metal-cyclodextrin complex according to the present invention is a complex of a metal selected from the group comprising or consisting of Ag, Zn, Cu, Pt, Au, Ru, As, Sb, Bi, Ti, V, Ni, Hg, Pb, Co, oxides, hydroxides and salts thereof; and a cyclodextrin of formula (I), wherein:
p is 6 or 7;
R is -OH or -X-NH-Z-NHR₁;
X is a single bond;
Z is -C=S;
Ri is selected from H and optionally substituted short chain alkyls; preferably R₁ is selected from methyl, ethyl propyl and butyl;
with the proviso one, two, three R = -X-NH-Z-NHR₁.

In one embodiment, the metal-cyclodextrin complex according to the present invention is a complex of a metal selected from the group comprising or consisting of Ag, Zn, Cu, Pt, Au, As, oxides, hydroxides and salts thereof; and a cyclodextrin of formula (I), wherein:
p is 6, 7 or 8;
R is -OH or -X-NH-Z-NHR₁;
X is a single bond or R₂;
Z is selected from -C=O and -C=S;
Ri is selected from H and optionally substituted short chain alkyls; preferably R₁ is selected from methyl, ethyl propyl and butyl;
R₂ is selected from optionally substituted short chain alkyls; preferably R₂ is selected from methyl, ethyl and propyl;
with the proviso that at least one R = -X-NH-Z-NHR₁.

In one embodiment, the metal-cyclodextrin complex according to the present invention is a complex of Ag, oxides, hydroxides or salts thereof; and a cyclodextrin of formula (I), wherein:
p is 6, 7 or 8;
R is -OH or -X-NH-Z-NHR₁;
X is a single bond or R₂;
Z is selected from -C=O and -C=S;
R₁ is selected from H and optionally substituted short chain alkyls; preferably R₁ is selected from methyl, ethyl propyl and butyl;
R₂ is selected from optionally substituted short chain alkyls; preferably R₂ is selected from methyl, ethyl and propyl;
with the proviso that at least one R = -X-NH-Z-NHR₁.

In one embodiment, the metal-cyclodextrin complex according to the present invention is a complex of Zn, oxides, hydroxides or salts thereof; and a cyclodextrin of formula (I), wherein:
p is 6, 7 or 8;
R is -OH or -X-NH-Z-NHR₁;
X is a single bond or R₂;
Z is selected from -C=O and -C=S;
R₁ is selected from H and optionally substituted short chain alkyls; preferably R₁ is selected from methyl, ethyl propyl and butyl;
R₂ is selected from optionally substituted short chain alkyls; preferably R₂ is selected from methyl, ethyl and propyl;
with the proviso that at least one R = -X-NH-Z-NHR₁.

In one embodiment, the metal-cyclodextrin complex according to the present invention is a complex of a metal selected from the group comprising or consisting of Ag, Zn, Cu, Pt, Au, As, oxides, hydroxides and salts thereof; and a cyclodextrin of formula (I), wherein:
p is 6 or 7;
R is -OH or -X-NH-Z-NHR₁;
X is a single bond or R₂;
Z is -C=S;
R₁ is selected from H and optionally substituted short chain alkyls; preferably R₁ is selected from methyl, ethyl propyl and butyl;
R₂ is selected from optionally substituted short chain alkyls; preferably R₂ is selected from methyl, ethyl and propyl;
with the proviso that at least one R = -X-NH-Z-NHR₁.

In one embodiment, the metal-cyclodextrin complex according to the present invention is a complex of a metal selected from the group comprising or consisting of Ag, Zn, Cu, Pt, Au, As, oxides, hydroxides and salts thereof; and a cyclodextrin of formula (I), wherein:
p is 6 or 7;
R is -OH or -X-NH-Z-NHR₁;
X is a single bond;
Z is -C=S;
R₁ is methyl;
with the proviso that at least one R = -X-NH-Z-NHR₁.

In one embodiment, the metal-cyclodextrin complex according to the present invention is a complex of a metal selected from the group comprising or consisting of Ag, Zn, oxides, hydroxides and salts thereof; and a cyclodextrin of formula (I), wherein:
p is 6 or 7;
R is -OH or -X-NH-Z-NHR₁;
X is a single bond;
Z is -C=S;
R₁ is methyl;
with the proviso that at least one R = -X-NH-Z-NHR₁.

In one embodiment, the metal-cyclodextrin complex according to the present invention is a complex of Ag, oxides, hydroxides or salts thereof; and a cyclodextrin of formula (I), wherein:
p is 6 or 7;
R is -OH or -X-NH-Z-NHR₁;
X is a single bond;
Z is -C=S;
R₁ is methyl;
with the proviso that at least one R = -X-NH-Z-NHR₁.

In one embodiment, the metal-cyclodextrin complex according to the present invention is a complex of Ag, oxides, hydroxides or salts thereof; and a cyclodextrin of formula (I), wherein:
p is 6 or 7;
R is -OH or -X-NH-Z-NHR₁;
X is a single bond;
Z is -C=S;
R₁ is methyl;
with the proviso that one R = -X-NH-Z-NHR₁.

In one embodiment, the metal-cyclodextrin complex according to the present invention is a complex of Ag salts; and a cyclodextrin of formula (I), wherein:
p is 6 or 7;
R is -OH or -X-NH-Z-NHR₁;
X is a single bond;
Z is -C=S;
R₁ is methyl;
with the proviso that one R = -X-NH-Z-NHR₁.

In one embodiment, the metal-cyclodextrin complex according to the present invention is a complex of AgNO₃; and a cyclodextrin of formula (I), wherein:
p is 6 or 7, preferably p is 7;
R is -OH or -X-NH-Z-NHR₁;
X is a single bond;
Z is -C=S;
R₁ is methyl;
with the proviso that one R = -X-NH-Z-NHR₁.

In one embodiment, the metal-cyclodextrin complex according to the present invention is a complex of Zn, oxides, hydroxides or salts thereof; and a cyclodextrin of formula (I), wherein:
p is 6 or 7;
R is -OH or -X-NH-Z-NHR₁;
X is a single bond;
Z is -C=S;
R₁ is methyl;
with the proviso that at least one R = -X-NH-Z-NHR₁.

In one embodiment, the metal-cyclodextrin complex according to the present invention is a complex of Zn, oxides, hydroxides or salts thereof; and a cyclodextrin of formula (I), wherein:
p is 6 or 7;
R is -OH or -X-NH-Z-NHR₁;
X is a single bond;
Z is -C=S;
R₁ is methyl;
with the proviso that two R = -X-NH-Z-NHR₁.

In one embodiment, the metal-cyclodextrin complex according to the present invention is a complex of Zn, oxides, hydroxides or salts thereof; and a cyclodextrin of formula (I), wherein:
p is 6 or 7;
R is -OH or -X-NH-Z-NHR₁;
X is a single bond;
Z is -C=S;
R₁ is methyl;
with the proviso that one R = -X-NH-Z-NHR₁.

In one embodiment, the metal-cyclodextrin complex according to the present invention is a complex of a metal selected from the group comprising or consisting of Ag, Zn, Cu, Pt, Au, As, oxides, hydroxides and salts thereof; and a β-cyclodextrin of formula (I), wherein:
p is 7;
R is -OH or -X-NH-Z-NHR₁;
X is a single bond or R₂;
Z is -C=S;
R₁ is selected from H and optionally substituted short chain alkyls; preferably R₁ is selected from methyl, ethyl propyl and butyl;
R₂ is selected from optionally substituted short chain alkyls; preferably R₂ is selected from methyl, ethyl and propyl;
with the proviso that at least one R = -X-NH-Z-NHR₁.

In one embodiment, the metal-cyclodextrin complex according to the present invention is a complex of a metal selected from the group comprising or consisting of Ag, Zn, Cu, Pt, Au, As, oxides, hydroxides and salts thereof; and a β-cyclodextrin of formula (I), wherein:
p is 7;
R is -OH or -X-NH-Z-NHR₁;
X is a single bond;
Z is -C=S;
R₁ is selected from H and methyl;
with the proviso that at least one R = -X-NH-Z-NHR₁.

In one embodiment, the metal-cyclodextrin complex according to the present invention is a complex of a metal selected from the group comprising or consisting of Ag, Zn, Cu, Pt, Au, As, oxides, hydroxides and salts thereof; and a β-cyclodextrin of formula (I), wherein:
p is 7;
R is -OH or -X-NH-Z-NHR₁;
X is a single bond;
Z is -C=S;
R₁ is methyl;
with the proviso that at least one R = -X-NH-Z-NHR₁.

In one embodiment, the metal-cyclodextrin complex according to the present invention is a complex of Ag, oxides, hydroxides or salts thereof; and a β-cyclodextrin of formula (I), wherein:
p is 7;
R is -OH or -X-NH-Z-NHR₁;
X is a single bond;
Z is -C=S;
R₁ is methyl;
with the proviso that at least one R = -X-NH-Z-NHR₁.

In one embodiment, the metal-cyclodextrin complex according to the present invention is a complex of Zn, oxides, hydroxides or salts thereof; and a β-cyclodextrin of formula (I), wherein:
p is 7;
R is -OH or -X-NH-Z-NHR₁;
X is a single bond;
Z is -C=S;
R₁ is methyl;
with the proviso that at least one R = -X-NH-Z-NHR₁.

In one embodiment, the metal-cyclodextrin complexes of the present invention can further comprise at least one therapeutic agent. The present invention can be implemented with any therapeutic agent known in the art.

In a preferred embodiment, the at least one therapeutic agent is an organic therapeutic agent. Therefore, in one embodiment, the metal-cyclodextrin complexes of the present invention can further comprise at least one organic therapeutic agent.

In one embodiment, the at least one organic therapeutic agent is selected from antibiotic, anti-fungal, antiparasitic, antiviral, anti-ulcer, anticancer, antidiabetic, anti-depressive and immune-modulating agents; salts thereof and combinations thereof.

In one embodiment, the at least one organic therapeutic agent is selected from antibiotic, anti-fungal, antiparasitic and antiviral agents; salts thereof and combinations thereof.

In one embodiment, the at least one organic therapeutic agent is selected from antibiotic agents, salts thereof and combinations thereof. In one embodiment, the at least one organic therapeutic agent is selected from anti-fungal agents, salts thereof and combinations thereof. In one embodiment, the at least one organic therapeutic agent is selected from antiviral agents, salts thereof and combinations thereof. In one embodiment, the at least one organic therapeutic agent is selected from antiparasitic agents, salts thereof and combinations thereof.

According to a first embodiment, the at least one organic therapeutic agent is selected from antibiotic agents, salts thereof and combinations thereof.

In one embodiment, the at least one organic therapeutic agent is an antibiotic agent selected from the group comprising or consisting of sulfamides, aminoglycosides, tetracyclines, oxazolidinones, amphenicols, pleuromutilins, macrolides, lincosamides, streptogramins, fusidic acid, fosfomycin, cycloserine, bacitracin; vancomycin; oritavancin, telavancin, teicoplanin; dalbavancin; ramoplanin, penicillins, penems, carbapenems, cephalosporins, monobactams, beta-lactamase inhibitors, polymyxins, diaminopyridines, dapsone, quinolones, nitro-imidazoles, nitrofurans, rifamycins, salts thereof and combinations thereof.

In one embodiment, the antibiotic agent is selected from the group comprising or consisting of
- sulfamides, selected from sulfadiazine, sulfaisodimidine, sulfamethizole, sulfadimidine, sulfapyridine, sulfafurazole, sulfanilamide, sulfathiazole, sulfathiourea, sulfamethoxazole, sulfamoxole, sulfadimethoxine, sulfadoxine, sulfalene, sulfametomidine, sulfametoxydiazine, sulfamethoxypyridazine, sulfaperin, sulfamerazine, sulfaphenazole, sulfamazone, sulfacetamide, sulfadicramide, sulfametrole and sulfanitran;
- aminoglycosides, selected from streptomycin, dihydrostreptomycin, neomycin, kanamycin, amikacin, arbekacin, bekanamycin, dibekacin, tobramycin, spectinomycin, hygromycin B, paromomycin, gentamicin, verdamicin and astromicin;
- tetracyclines selected from doxycycline, chlortetracycline, clomocycline, demeclocycline, lymecycline, meclocycline, metacycline, minocycline, omadacycline, oxytetracycline, penimepicycline, rolitetracycline, sarecycline and tetracycline;
- oxazolidinones selected from eperezolid, linezolid, posizolid, radezolid, ranbezolid, sutezolid and tedizolid;
- amphenicols selected from, chloramphenicol, azidamfenicol, thiamphenicol and florfenicol;
- pleuromutilins selected from retapamulin, tiamulin and valnemulin;
- macrolides selected from azithromycin, clarithromycin, dirithromycin, erythromycin, flurithromycin, josamycin, midecamycin, miocamycin, oleandomycin, rokitamycin, roxithromycin, spiramycin, troleandomycin, tylosin, telithromycin, cethromycin and solithromycin;
- lincosamides selected from clindamycin, lincomycin and pirlimycin;
- streptogramins selected from pristinamycin, quinupristin, dalfopristin and virginiamycin;
- fusidic acid;
- fosfomycin;
- cycloserine;
- bacitracin;
- vancomycin;
- oritavancin;
- telavancin;
- teicoplanin;
- dalbavancin;
- ramoplanin;
- penicillins selected from benzylpenicillin G, benzathine benzylpenicillin, procaine benzylpenicillin, phenoxymethylpenicillin V, propicillin, pheneticillin, azidocillin, clometocillin, penamecillin, cloxacillin, dicloxacillin, flucloxacillin, oxacillin, nafcillin, methicillin, amoxicillin, pivampicillin, ampicillin, hetacillin, bacampicillin, metampicillin, talampicillin, ppicillin, ticarcillin, carbenicillin, carindacillin, temocillin, piperacillin, azlocillin, mezlocillin, mecillinam, pivmecillinam and sulbenicillin;
- penems selected from faropenem and ritipenem;
- carbapenems selected from ertapenem, doripenem, imipenem, meropenem, biapenem and panipenem;
- cephalosporins selected from cefazolin, cefalexin cefadroxil, cefapirin, cefazedone, cefazaflur, cefradine, cefroxadine, ceftezole, cefaloglycin, cefacetrile, cefalonium, cefaloridine, cefalotin, cefatrizine, cefaclor, cefotetan, cephamycin, cefoxitin, cefprozil, cefuroxime, cefuroxime axetil, cefamandole, cefminox, cefonicid, ceforanide, cefotiam, cefbuperazone, cefuzonam, cefmetazole, carbacephem, cefixime, ceftriaxone, ceftazidime, cefoperazone, cefdinir, cefcapene, cefdaloxime, ceftizoxime, cefmenoxime, cefotaxime, cefpiramide, cefpodoxime, ceftibuten, cefditoren, cefetamet, cefodizime, cefpimizole, cefsulodin, cefteram, ceftiolene, oxacephem, fomoxef, latamoxef, cefepime, cefozopran, cefpirome, cefquinome, ceftaroline, fosamil, ceftolozane, ceftobiprole, ceftiofur, cefquinome and cefovecin;
- monobactams selected from aztreonam, tigemonam, carumonam and nocardicin A;
- beta-lactamase inhibitors selected from sulbactam, tazobactam, clavulanic acid, avibactam and vaborbactam;
- polymyxins selected from polysporin and neosporin;
- daptomycin;
- tyrothricin;
- gramicidin tyrocidine;
- isoniazid;
- teixobactin;
- 2,4-diaminopyridines selected from trimethoprim, brodimoprim, tetroxoprim, iclaprim and ormetoprim;
- dapsone;
- quinolones selected from cinoxacin, flumequine, nalidixic acid, oxolinic acid, pipemidic acid, piromidic acid, rosoxacin, ciprofloxacin, ofloxacin, enoxacin, fleroxacin, lomefloxacin, nadifloxacin, norfloxacin, pefloxacin, rufloxacin,, levofloxacin, balofloxacin, grepafloxacin, pazufloxacin, sparfloxacin, temafloxacin, tosufloxacin, besifloxacin, delafloxacin, gatifloxacin, finafloxacin, gemifloxacin, moxifloxacin, clinafloxacin, garenoxacin, prulifloxacin, sitafloxacin, trovafloxacin, alatrofloxacin, danofloxacin difloxacin, enrofloxacin, ibafloxacin, marbofloxacin, orbifloxacin, pradofloxacin, sarafloxacin, nemonoxacin and novobiocin;
- nitro-imidazoles selected from metronidazole, tinidazole, mebendazole, ornidazole;
- nitrofurans selected from nitrofurantoin, furazolidone and nifurtoinol;
- rifamycins selected from rifampicin, rifabutin, rifapentine, rifaximin rifalazil;
- mupirocin;
- clofoctol;
- xibornol;
- nitroxoline;
- salts thereof;
- and combinations thereof.

In one embodiment, the antibiotic agent is selected from the group comprising or consisting of sulfamides, aminoglycosides, tetracyclines, oxazolidinones, amphenicols, pleuromutilins, macrolides, lincosamides, streptogramins, fusidic acid, fosfomycin, cycloserine, bacitracin, vancomycin, oritavancin, telavancin, teicoplanin, dalbavancin, ramoplanin, penicillins, penems, carbapenems, cephalosporins, monobactams, beta-lactamase inhibitors, salts thereof and combinations thereof.

In one embodiment, the antibiotic agent is selected from the group comprising or consisting of sulfamides, aminoglycosides, tetracyclines, amphenicols, macrolides, fusidic acid, bacitracin, penicillins, penems, carbapenems, cephalosporins, beta-lactamase inhibitors, diaminopyridines, quinolones, nitro-imidazoles, nitrofurans, rifamycins, salts thereof and combinations thereof.

In one embodiment, the antibiotic agent is selected from the group comprising or consisting of sulfamides, salts thereof and combinations thereof.

In one embodiment, the antibiotic agent is a sulfamide selected from the group comprising or consisting of sulfadiazine, sulfaisodimidine, sulfamethizole, sulfadimidine, sulfapyridine, sulfafurazole, sulfanilamide, sulfathiazole, sulfathiourea, sulfamethoxazole, sulfamoxole, sulfadimethoxine, sulfadoxine, sulfalene, sulfametomidine, sulfametoxydiazine, sulfamethoxypyridazine, sulfaperin, sulfamerazine, sulfaphenazole, sulfamazone, sulfacetamide, sulfadicramide, sulfametrole and sulfanitran, salts thereof and combinations thereof.

In one embodiment, the antibiotic is sulfadiazine, salts thereof or combinations thereof.

In one embodiment, the at least one organic therapeutic agent is an antibiotic agent selected from the group comprising or consisting of aminoglycosides, salts thereof and combinations thereof. In one embodiment, the at least one organic therapeutic agent is an antibiotic agent selected from the group comprising or consisting of tetracyclines, salts thereof and combinations thereof. In one embodiment, the at least one organic therapeutic agent is an antibiotic agent selected from the group comprising or consisting of amphenicols, salts thereof and combinations thereof. In one embodiment, the at least one organic therapeutic agent is an antibiotic agent selected from the group comprising or consisting of macrolides, salts thereof and combinations thereof. In one embodiment, the at least one organic therapeutic agent is an antibiotic agent selected from the group comprising or consisting of fusidic acid, salts thereof and combinations thereof. In one embodiment, the at least one organic therapeutic agent is an antibiotic agent selected from the group comprising or consisting of bacitracin, salts thereof and combinations thereof. In one embodiment, the at least one organic therapeutic agent is an antibiotic agent selected from the group comprising or consisting of penicillins, salts thereof and combinations thereof. In one embodiment, the at least one organic therapeutic agent is an antibiotic agent selected from the group comprising or consisting of penems and carbapenems, salts thereof and combinations thereof. In one embodiment, the at least one organic therapeutic agent is an antibiotic agent selected from the group comprising or consisting of cephalosporins, salts thereof and combinations thereof. In one embodiment, the at least one organic therapeutic agent is an antibiotic agent selected from the group comprising or consisting of beta-lactamase inhibitors, salts thereof and combinations thereof. In one embodiment, the at least one organic therapeutic agent is an antibiotic agent selected from the group comprising or consisting of diaminopyridines, salts thereof and combinations thereof. In one embodiment, the at least one organic therapeutic agent is an antibiotic agent selected from the group comprising or consisting of quinolones, salts thereof and combinations thereof. In one embodiment, the at least one organic therapeutic agent is an antibiotic agent selected from the group comprising or consisting of nitro-imidazoles, salts thereof and combinations thereof. In one embodiment, the at least one organic therapeutic agent is an antibiotic agent selected from the group comprising or consisting of nitrofurans, salts thereof and combinations thereof. In one embodiment, the at least one organic therapeutic agent is an rifamycins, salts thereof and combinations thereof.

According to a second embodiment, the at least one organic therapeutic agent is selected from antifungal agents, salts thereof and combinations thereof.

In one embodiment, the at least one organic therapeutic agent is an antifungal agent selected from the group comprising or consisting of
- pyrithione;
- imidazoles selected from bifonazole, butoconazole, chlormidazole, clotrimazole, croconazole, eberconazole, econazole, fenticonazole, flutrimazole, isoconazole, ketoconazole, luliconazole, miconazole, neticonazole, omoconazole, oxiconazole, sertaconazole, sulconazole, tioconazole;
- triazoles selected from efinaconazole, fluconazole, fosfluconazole, terconazole, hexaconazole, isavuconazole, itraconazole, posaconazole, voriconazole, albaconazole, ravuconazole;
- abafungin;
- polyenes selected from natamycin, nystatin, amphotericin B and hamycin;
- allylamines selected from naftifine and terbinafine;
- amorfoline;
- echinocandins selected from anidulafungin, biafungin, caspofungin, cilofungin and micafungin;
- frucytosine;
- griseofluvin;
- tavaborole;
- bromochlorosalicylanilide;
- chlorophetanol;
- chlorphenesin; ciclopirox; dimazole;
- ethylparaben;
- haloprogin;
- polynoxylin;
- salicylic acid;
- salicylic acid methyl-ester;
- sulbentine;
- taurolidine;
- ticlatone;
- tolciclate;
- tolnaftate;
- tribromometacresol;
- undecylenic acid;
- atovaquone;
- dapsone;
- pentamidine;
- fumagillin;
- essential oils selected from citronella oil, lemongrass oil, lemon myrtle oil, orange peel oil, patchouli oil and tea tree oil;
- salts thereof; and
- combinations thereof.

In one embodiment, the at least one antifungal agent is selected from the group comprising or consisting of pyrithione, imidazoles, triazoles, polyenes, griseofluvin, bromochlorosalicylanilide, chlorophetanol, ciclopirox, dimazole, salicylic acid methyl-ester, tolnaftate, tribromometacresol, undecylenic acid, atovaquone, dapsone, fumagillin, essential oils, salts thereof and combinations thereof. In one embodiment, the at least one antifungal agent is selected from the group comprising or consisting of pyrithione, imidazoles, triazoles, polyenes, griseofluvin, chlorophetanol, ciclopirox, dimazole, salicylic acid methyl-ester, tolnaftate, tribromometacresol, atovaquone, dapsone, essential oils, salts thereof and combinations thereof.

In one embodiment, the at least one antifungal agent is pyrithione, salts thereof or combinations thereof.

In one embodiment, the at least one antifungal agent is selected from the group comprising or consisting of imidazoles, salts thereof and combinations thereof.

In one embodiment, the at least one antifungal agent is selected from the group comprising or consisting of triazoles, salts thereof and combinations thereof.

According to a third embodiment, the at least one organic therapeutic agent is selected from antiviral agents, salts thereof and combinations thereof.

In one embodiment, the at least one organic therapeutic agent is an antiviral agent selected from the group comprising or consisting of acyclovir, docosanol, ganciclovir, imiquimod, penciclovir, podofilox and podophyllin resin.

According to a fourth embodiment, the at least one organic therapeutic agent is selected from antiparasitic agents, salts thereof and combinations thereof.

In one embodiment, the at least one organic therapeutic agent is an antiparasitic agent selected from the group comprising or consisting of
- nitro-imidazoles selected from metronidazole, tinidazole, mebendazole and ornidazole;
- eflornithine;
- furazolidone;
- melarsoprol;
- nifursemizone;
- nitazoxanide;
- ornidazole;
- paromomycin;
- pentamidine;
- pyrimethamine;
- chloroquine;
- clotrimazole;
- crotamiton;
- benzyl benzoate;
- ivermectin;
- thiabendazole;
- diethylcarbamazine;
- niclosamide;
- praziquantel;
- miltefosine;
- salts thereof; and
- combinations thereof.

In one embodiment, the at least one antiparasitic agent is selected from the group comprising or consisting of nitro-imidazoles selected from metronidazole, tinidazole, mebendazole and ornidazole; eflornithine; furazolidone; melarsoprol; nifursemizone; nitazoxanide; ornidazole; paromomycin; pentamidine; pyrimethamine; chloroquine; clotrimazole; crotamiton; benzyl benzoate; ivermectin; thiabendazole, diethylcarbamazine; niclosamide; praziquantel; miltefosine; salts thereof, and combinations thereof. In one embodiment, the at least one antiparasitic agent is selected from the group comprising or consisting of eflornithine, salts thereof and combinations thereof. In one embodiment, the at least one antiparasitic agent is selected from the group comprising or consisting of furazolidone, salts thereof and combinations thereof. In one embodiment, the at least one antiparasitic agent is selected from the group comprising or consisting of melarsoprol, salts thereof, and combinations thereof. In one embodiment, the at least one antiparasitic agent is selected from the group comprising or consisting of nifursemizone, salts thereof, and combinations thereof. In one embodiment, the at least one antiparasitic agent is selected from the group comprising or consisting of nitazoxanide, salts thereof, and combinations thereof. In one embodiment, the at least one antiparasitic agent is selected from the group comprising or consisting of pyrimethamine salts thereof, and combinations thereof. In one embodiment, the at least one antiparasitic agent is selected from the group comprising or consisting of chloroquine, salts thereof, and combinations thereof. In one embodiment, the at least one antiparasitic agent is selected from the group comprising or consisting of clotrimazole, salts thereof, and combinations thereof. In one embodiment, the at least one antiparasitic agent is selected from the group comprising or consisting of crotamiton, salts thereof, and combinations thereof. In one embodiment, the at least one antiparasitic agent is benzyl benzoate.

In one embodiment, the at least one antiparasitic agent is selected from the group comprising or consisting of ivermectin, salts thereof, and combinations thereof.

In one embodiment, the at least one antiparasitic agent is selected from the group comprising or consisting of diethylcarbamazine, niclosamide, praziquantel, miltefosine, salts thereof, and combinations thereof.

Metal-organic therapeutic agent association consist in an effective therapeutic approach in a broad spectrum of pathologies comprising depression, circulatory disorders, hypercalcemia, hyperphosphatemia, arthrosis, gastric ulcer, cancer, microbial infections, fungal infections and parasitic infections (Farrell N., CCC II, 2013;9:809-840). Lack of sufficient aqueous solubility is one of the major obstacles impeding the wide use of existing and promising metal-organic therapeutic agent associations. The present invention offers the technology to overcome the obstacle of aqueous solubility.

Indicative embodiments of the invention consist in the association of any metal, as previously detailed, with at least one organic therapeutic agents, as previously detailed.

In one embodiment, the metal-organic therapeutic agent association comprises a metal selected from the group comprising or consisting of Ag, Zn, Cu, Pt, Au, Ru, As, Sb, Bi, Ti, V, Ni, Hg, Pb, Co, oxides, hydroxides and salts thereof; and an organic therapeutic agent selected from the group comprising or consisting of antibiotic, anti-fungal, antiviral, antiparasitic, anti-ulcer, anticancer, antidiabetic, anti-depressive, immune-modulating agents, salts thereof and combinations thereof.

Indicative and non-limitative embodiments of the present invention comprise:
- Ag oxides, hydroxides and salts thereof; with at least one antibiotic agent;
- Ag, oxides, hydroxides and salts thereof; with at least one antifungal agent;
- Zn, oxides, hydroxides and salts thereof; with at least one antibiotic agent;
- Zn, oxides, hydroxides and salts thereof; with at least one antifungal agent;
- Zn, oxides, hydroxides and salts thereof; with at least one antiviral agent;
- Cu, oxides, hydroxides and salts thereof; with at least one antibiotic agent;
- Cu, oxides, hydroxides and salts thereof; with at least one antifungal agent;
- Cu, oxides, hydroxides and salts thereof; with at least one antiviral agent;
- Pt, oxides, hydroxides and salts thereof; with at least one antiviral agent;
- Pt, oxides, hydroxides and salts thereof; with at least one anticancer agent;
- Au, oxides, hydroxides and salts thereof; with at least one immune-modulating agent;
- Au, oxides, hydroxides and salts thereof; with at least one antiarthritic agent;
- Ru, oxides, hydroxides and salts thereof; with at least one antiviral agent;
- As, oxides, hydroxides and salts thereof; with at least one antiparasitic agent;
- Sb, oxides, hydroxides and salts thereof; with at least one antiparasitic agent;
- Bi, oxides, hydroxides and salts thereof; with at least one antibiotic agent;
- Bi, oxides, hydroxides and salts thereof; with at least one anti-ulcer agent;
- V, oxides, hydroxides and salts thereof; with at least one antidiabetic agent;
- Ni, oxides, hydroxides and salts thereof; with at least one antiviral agent; and
- Co, oxides, hydroxides and salts thereof; with at least one antiviral agent.

In one embodiment, the metal-organic therapeutic agent association comprises a metal selected from the group comprising or consisting of Ag, Zn, Cu, Pt, Au, Ru, As, Sb, Bi, oxides, hydroxides and salts thereof; and an organic therapeutic agent selected from antibiotic, anti-fungal, antiviral and antiparasitic, agents, salts thereof and combinations thereof.

In one embodiment, the metal-organic therapeutic agent association comprises a metal selected from the group comprising or consisting of Ag, Zn, Cu, oxides, hydroxides and salts thereof; and an organic therapeutic agent selected from antibiotic, anti-fungal, antiviral and antiparasitic agents and combinations thereof.

In one embodiment, the metal-organic therapeutic agent association comprises a metal selected from the group comprising or consisting of Ag, Zn, Cu, oxides, hydroxides and salts thereof; and an organic therapeutic agent selected from antibiotic agents, salts thereof and combinations thereof.

In one embodiment, the metal-organic therapeutic agent association comprises a metal selected from the group comprising or consisting of Ag, Zn, Cu, oxides, hydroxides and salts thereof; and an organic therapeutic agent selected from anti-fungal, antiviral and antiparasitic agents, salts thereof and combinations thereof.

In one embodiment, the metal-organic therapeutic agent association comprises a metal selected from the group comprising or consisting of Ag, Zn, Cu, Pt, Au, Ru, As, Sb, Bi, Ti, V, Ni, Hg, Pb, Co, oxides, hydroxides and salts thereof; and an organic therapeutic agent selected from antibiotic, anti-fungal, antiviral, antiparasitic, anti-ulcer, anticancer, antidiabetic, anti-depressive, immune-modulating agents, salts thereof and combinations thereof.

In one embodiment, the metal-organic therapeutic agent association comprises a metal selected from the group comprising or consisting of Ag, Zn, Cu, Pt, Au, Ru, As, Sb, Bi, Ti, V, Ni, Hg, Pb, Co, oxides, hydroxides and salts thereof; and an organic therapeutic agent selected from antibiotic, anti-fungal, antiviral, antiparasitic, anti-ulcer, anticancer, antidiabetic, anti-depressive, immune-modulating agents, salts thereof and combinations thereof.

In one embodiment, the metal-organic therapeutic agent association comprises a metal selected from the group comprising or consisting of Ag, Zn, Cu, Pt, Au, oxides, hydroxides and salts thereof; and an organic therapeutic agent selected from antibiotic, anti-fungal, antiviral, antiparasitic, anti-ulcer, anticancer, antidiabetic, anti-depressive, immune-modulating agents, salts thereof and combinations thereof.

Metals and their dissolved ions, such as, e.g., silver, attacks bacterial cells by rendering the bacterial cell membrane more permeable, and interferes with the bacterial cell's metabolism, leading to the overproduction of reactive, toxic chemical species (Owens B. Nature News, June 19 2013). Such pharmacodynamic properties of metals can be implemented to make current antibiotics or antibiotics in development more effective against resistant bacteria.

According to a preferred embodiment, the metal-organic therapeutic agent association comprises or consists of Ag as a metal and an antibiotic agent as organic therapeutic agent, preferably the antibiotic agent is selected from sulfamides, more preferably the antibiotic agent is sulfadiazine.

According to a preferred embodiment, the metal-organic therapeutic agent association comprises or consists of Zn as a metal and an antifungal agent as organic therapeutic agent, preferably the antifungal agent is pyrithione.

As previously discussed, the metal-cyclodextrin complexes of the present invention can further comprise at least one therapeutic agent.

In one embodiment, the complex according to the present invention comprises or consists of
(i) a metal selected from the group comprising or consisting of Ag, Zn, Cu, Pt, Au, Ru, As, Sb, Bi, Ti, V, Ni, Hg, Pb, Co, oxides, hydroxides and salts thereof;
(ii) a cyclodextrin of formula (I) 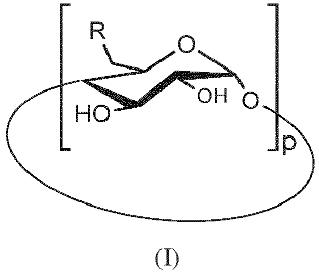 wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁; and
(iii) at least one organic therapeutic agent selected from the group comprising or consisting of antibiotic, anti-fungal, antiviral, antiparasitic, anti-ulcer, anticancer, antidiabetic, anti-depressive, immune-modulating agents, salts thereof and combinations thereof.

In one embodiment, the complex according to the present invention comprises or consists of
(i) a metal selected from the group comprising or consisting of Ag, Zn, Cu, Pt, Au, Ru, As, Sb, Bi, Ti, V, Ni, Hg, Pb, Co, oxides, hydroxides and salts thereof;
(ii) a cyclodextrin of formula (I), wherein:
   p is 6 or 7;
   R is -OH or -X-NH-Z-NHR₁;
   X is a single bond;
   Z is -C=S;
   R₁ is selected from H and optionally substituted short chain alkyls; preferably R₁ is selected from methyl, ethyl propyl and butyl;
   with the proviso that at least one R = -X-NH-Z-NHR₁; and
(iii) at least one organic therapeutic agent selected from the group comprising or consisting of antibiotic, anti-fungal, antiviral, antiparasitic, anti-ulcer, anticancer, antidiabetic, anti-depressive, immune-modulating agents, salts thereof and combinations thereof.

In one embodiment, the complex according to the present invention comprises or consists of
(i) a metal selected from the group comprising or consisting of Ag, Zn, Cu, Pt, Au, As, oxides, hydroxides and salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁; and
(iii) at least one organic therapeutic agent selected from the group comprising or consisting of antibiotic, anti-fungal, antiviral, antiparasitic agents, salts thereof and combinations thereof.

In one embodiment, the complex according to the present invention comprises or consists of
(i) a metal selected from the group comprising or consisting of Ag, Zn, Cu, Pt, Au, oxides, hydroxides and salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁; and
(iii) at least one organic therapeutic agent selected from the group comprising or consisting of antibiotic, anti-fungal, antiviral, antiparasitic agents, salts thereof and combinations thereof.

In one embodiment, the complex according to the present invention comprises or consists of
(i) Ag, oxides, hydroxides or salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁; and
(iii) at least one organic therapeutic agent selected from the group comprising or consisting of antibiotic, anti-fungal, antiviral, antiparasitic agents, salts thereof and combinations thereof.

In one embodiment, the complex according to the present invention comprises or consists of
(i) Ag, oxides, hydroxides and salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁; and
(iii) at least one organic therapeutic agent selected from the group comprising or consisting of antibiotic agents, salts thereof and combinations thereof.

In one embodiment, the complex according to the present invention comprises or consists of
(i) Ag, oxides, hydroxides or salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁; and
(iii) at least one organic therapeutic agent selected from the group comprising or consisting of sulfonamides, salts thereof and combinations thereof.

In one embodiment, the complex according to the present invention comprises or consists of
(i) Ag, oxides, hydroxides or salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁; and
(iii) sulfadiazine.

In one embodiment, the complex according to the present invention comprises or consists of
(i) Zn, oxides, hydroxides and salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) at least one organic therapeutic agent selected from the group comprising or consisting of antibiotic, anti-fungal, antiviral, antiparasitic agents, salts thereof and combinations thereof.

In one embodiment, the complex according to the present invention comprises or consists of
(i) Zn, oxides, hydroxides and salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) at least one organic therapeutic agent selected from the group comprising or consisting of antibiotic, anti-fungal, antiviral, antiparasitic agents, salts thereof and combinations thereof.

In one embodiment, the complex according to the present invention comprises or consists of
(i) Ag, oxides, hydroxides and salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) at least one organic therapeutic agent selected from the group comprising or consisting of antibiotic and anti-fungal agents, salts thereof and combinations thereof.

In one embodiment, the complex according to the present invention comprises or consists of
(i) Ag, oxides, hydroxides and salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) at least one organic therapeutic agent selected from the group comprising or consisting of anti-fungal agents, salts thereof and combinations thereof.

In one embodiment, the complex according to the present invention comprises or consists of
(i) Ag, oxides, hydroxides and salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) sulfadiazine.

In one embodiment, the complex according to the present invention comprises or consists of
(i) Ag;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) sulfadiazine.

In one embodiment, the complex according to the present invention comprises or consists of
(i) Ag;
(ii) a cyclodextrin of formula (I), wherein:
   p is 7;
   R is -OH or -X-NH-Z-NHR₁;
   X is a single bond;
   Z is -C=S;
   R₁ methyl;
   with the proviso that at least one R = -X-NH-Z-NHR₁;
(iii) sulfadiazine.

Advantageously, the metal-organic therapeutic agent-cyclodextrin complexes of the present invention maintain substantially the same molar ratio between the organic therapeutic agent and the metal as compared to non-complexed metal-organic therapeutic agent associations.

The molar ratio of between the organic therapeutic agent and the metal in metal-organic therapeutic agent associations depends on the valence of the metal and the electron-donor moieties of the organic therapeutic agent. For example, in the case of silver sulfadiazine, this ratio is 1; and in the case of zinc pyrithione, this ratio is 2.

In one embodiment, the molar ratio between the cyclodextrin-complexed organic therapeutic agent and the cyclodextrin-complexed metal is ranging from about 200 to 1, from about 150 to 1, from about 100 to 1, from about 50 to 1, from about 20 to 1, from about 15 to 1, from about 10 to 1, from about 9 to 1, from about 8 to 1, from about 7 to 1, from about 6 to 1, from about 5 to 1, from about 4 to 1, from about 3 to 1, from about 2 to 1, from about 1.5 to 1.

In one embodiment, the molar ratio between the cyclodextrin-complexed organic therapeutic agent and the complexed metal is about 1, about 1.5, about 2, about 3, about 4, about 5, about 6, about 7, about 8, about 9, about 10, about 12, about 14, about 16, about 18, about 20, about 25, about 30, about 35, about 40, about 50, about 60, about 80, about 100, about 120, about 140, about 150, about 200.

In one embodiment, the complex according to the invention comprises or consists of
(i) a metal selected from the group comprising or consisting of Ag, Zn, Cu, Pt, Au, Ru, As, Sb, Bi, Ti, V, Ni, Hg, Pb, Co, oxides, hydroxides and salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁; and
(iii) at least one organic therapeutic agent selected from antibiotic, anti-fungal, antiviral, antiparasitic, anti-ulcer, anticancer, antidiabetic, anti-depressive, immune-modulating agents, salts thereof and combinations thereof;
wherein the molar ratio between the complexed organic therapeutic agent and the complexed metal is ranging from about 200 to 1, from about 150 to 1, from about 100 to 1, from about 50 to 1, from about 20 to 1, from about 15 to 1, from about 10 to 1, from about 9 to 1, from about 8 to 1, from about 7 to 1, from about 6 to 1, from about 5 to 1, from about 4 to 1, from about 3 to 1, from about 2 to 1, from about 1.5 to 1.

In one embodiment, the complex according to the invention comprises or consists of
(i) a metal selected from the group comprising or consisting of Ag, Zn, Cu, Pt, Au, Ru, As, Sb, Bi, Ti, V, Ni, Hg, Pb, Co, oxides, hydroxides and salts thereof;
(ii) a cyclodextrin of formula (I), wherein:
   p is 6 or 7;
   R is -OH or -X-NH-Z-NHR₁;
   X is a single bond;
   Z is -C=S; R₁ is selected from H and optionally substituted short chain alkyls;
   preferably R₁ is selected from methyl, ethyl propyl and butyl;
   with the proviso that at least one R = -X-NH-Z-NHR₁; and
(iii) at least one organic therapeutic agent selected from antibiotic, anti-fungal, antiviral, antiparasitic, anti-ulcer, anticancer, antidiabetic, anti-depressive, immune-modulating agents, salts thereof and combinations thereof;
wherein the molar ratio between the complexed organic therapeutic agent and the complexed metal is ranging from about 20 to 1, from about 15 to 1, from about 10 to 1, from about 9 to 1, from about 8 to 1, from about 7 to 1, from about 6 to 1, from about 5 to 1, from about 4 to 1, from about 3 to 1, from about 2 to 1, from about 1.5 to 1.

In one embodiment, the complex according to the invention comprises or consists of
(i) a metal selected from the group comprising or consisting of Ag, Zn, Cu, Pt, Au, As, oxides, hydroxides and salts thereof;
(ii) a cyclodextrin of formula (I), wherein:
   p is 6, 7 or 8;
   R is -OH or -X-NH-Z-NHRi;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) the metal complex further comprising at least one organic therapeutic agent selected from antibiotic, anti-fungal, antiviral, antiparasitic agents, salts thereof and combinations thereof;
wherein the molar ratio between the complexed organic therapeutic agent and the complexed metal is ranging from about 200 to 1, from about 150 to 1, from about 100 to 1, from about 50 to 1, from about 20 to 1, from about 15 to 1, from about 10 to 1, from about 9 to 1, from about 8 to 1, from about 7 to 1, from about 6 to 1, from about 5 to 1, from about 4 to 1, from about 3 to 1, from about 2 to 1, from about 1.5 to 1.

In one embodiment, the complex according to the invention comprises or consists of
(i) a metal selected from the group comprising or consisting of Ag, Zn, Cu, Pt, Au, oxides, hydroxides and salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) at least one organic therapeutic agent selected from antibiotic, anti-fungal, antiviral, antiparasitic agents, salts thereof and combinations thereof;
wherein the molar ratio between the complexed organic therapeutic agent and the complexed metal is ranging from about 20 to 1, from about 15 to 1, from about 10 to 1, from about 9 to 1, from about 8 to 1, from about 7 to 1, from about 6 to 1, from about 5 to 1, from about 4 to 1, from about 3 to 1, from about 2 to 1, from about 1.5 to 1.

In one embodiment, the complex according to the invention comprises or consists of
(i) Ag, oxides, hydroxides and salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) at least one organic therapeutic agent selected from antibiotic, anti-fungal, antiviral, antiparasitic agents, salts thereof and combinations thereof;
wherein the molar ratio between the complexed organic therapeutic agent and the complexed Ag, oxides, hydroxides and salts thereof is ranging from about 200 to about 1, preferably from about 20 to about 2, even more preferably from about 2 to 1.

In one embodiment, the complex according to the invention comprises or consists of
(i) Ag, oxides, hydroxides and salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) at least one organic therapeutic agent selected from antibiotic agents, salts thereof and combinations thereof;
wherein the molar ratio between the complexed organic therapeutic agent and the complexed Ag, oxides, hydroxides and salts thereof is ranging from about 200 to about 1, preferably from about 20 to about 2, even more preferably from about 2 to 1.

In one embodiment, the complex according to the invention comprises or consists of
(i) Ag, oxides, hydroxides and salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) at least one organic therapeutic agent selected from sulfonamides, salts thereof and combinations thereof;
wherein the molar ratio between the complexed organic therapeutic agent and the complexed Ag, oxides, hydroxides and salts thereof is ranging from about 200 to about 1, preferably from about 20 to about 2, even more preferably from about 2 to 1.

In one embodiment, the complex according to the invention comprises or consists of
(i) Ag, oxides, hydroxides and salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) sulfadiazine;
wherein the molar ratio between the complexed sulfadiazine and the complexed Ag, oxides, hydroxides and salts thereof is ranging from about 200 to about 1, preferably from about 20 to about 2, even more preferably from about 2 to 1.

In one embodiment, the complex according to the invention comprises or consists of
(i) Zn, oxides, hydroxides and salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) at least one organic therapeutic agent selected from antibiotic, anti-fungal, antiviral, antiparasitic agents, salts thereof and combinations thereof;
wherein the molar ratio between the complexed organic therapeutic agent and the complexed Zn, oxides, hydroxides and salts thereof is ranging from about 200 to about 1, preferably from about 20 to about 2, even more preferably is about 2.

In one embodiment, the complex according to the invention comprises or consists of
(i) Zn, oxides, hydroxides and salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) at least one organic therapeutic agent selected from antibiotic, anti-fungal, antiviral, antiparasitic agents, salts thereof and combinations thereof;
wherein the molar ratio between the complexed organic therapeutic agent and the complexed Zn, oxides, hydroxides and salts thereof is ranging from about 200 to about 1, preferably from about 20 to about 2, even more preferably is about 2.

In one embodiment, the complex according to the invention comprises or consists of
(i) Ag, oxides, hydroxides and salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) at least one organic therapeutic agent selected from antibiotic and anti-fungal agents, salts thereof and combinations thereof;
wherein the molar ratio between the complexed organic therapeutic agent and the complexed Ag, oxides, hydroxides and salts thereof is ranging from about 200 to about 1, preferably from about 20 to about 2, even more preferably is about 1.

In one embodiment, the complex according to the invention comprises or consists of
(i) Ag, oxides, hydroxides and salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) at least one organic therapeutic agent selected from anti-fungal agents, salts thereof and combinations thereof;
wherein the molar ratio between the complexed organic therapeutic agent and the complexed Ag, oxides, hydroxides and salts thereof is ranging from about 200 to about 1, preferably from about 20 to about 2, even more preferably from about 2 to 1

In one embodiment, the complex according to the invention comprises or consists of
(i) Ag, oxides, hydroxides and salts thereof;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) sulfadiazine;
wherein the molar ratio between the complexed sulfadiazine and the complexed Ag, oxides, hydroxides and salts thereof is ranging from about 200 to about 1, preferably from about 20 to about 2, even more preferably from about 2 to 1.

In one embodiment, the complex according to the invention comprises or consists of
(i) Ag;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) sulfadiazine;
wherein the molar ratio between the complexed sulfadiazine and the complexed Ag is ranging from about 200 to about 1, preferably from about 20 to about 2, even more preferably from about 2 to 1.

In one embodiment, the complex according to the invention comprises or consists of
(i) Ag;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) sulfadiazine;
wherein the molar ratio between the complexed sulfadiazine and the complexed Ag is ranging from about 20 to about 2, preferably from about 2 to 1, even more preferably from about 1.5 to 1.

In one embodiment, the complex according to the invention comprises or consists of
(i) Ag;
(ii) a cyclodextrin of formula (I), wherein:
   p is 6, 7 or 8;
   R is -OH or -X-NH-Z-NHR₁;
   X is a single bond or R₂;
   Z is selected from -C=O and -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁;
(iii) sulfadiazine;
wherein the molar ratio between the complexed sulfadiazine and the complexed Ag is ranging from about 1.5 to 1.

In one embodiment, the complex according to the invention comprises or consists of
(i) Ag;
(ii) a cyclodextrin of formula (I), wherein:
   p is 7;
   R is -OH or -X-NH-Z-NHR₁;
   X is a single bond;
   Z is -C=S;
   R₁ methyl;
   with the proviso that at least one R = -X-NH-Z-NHR₁;
(iii) sulfadiazine;
wherein the molar ratio between the complexed sulfadiazine and the complexed Ag is ranging from about 20 to about 2, preferably from about 2 to 1, even more preferably from about 1.5 to 1.

In one embodiment, the complex according to the invention comprises or consists of
(i) Ag;
(ii) a cyclodextrin of formula (I), wherein:
   p is 7;
   R is -OH or -X-NH-Z-NHR₁;
   X is a single bond;
   Z is -C=S; Ri methyl;
   with the proviso that at least one R = -X-NH-Z-NHR₁;
(iii) sulfadiazine;
wherein the molar ratio between the complexes sulfadiazine and the complexed Ag is ranging from about 1.5 to 1.

The present invention further relates to compositions, preferably aqueous compositions comprising the complexes of the invention as previously described.

In one embodiment, the compositions according to the invention are aqueous solutions.

In one embodiment, the compositions are oil-in-water emulsions. In one embodiment, the compositions are water-in-oil-in-water emulsions.

In one embodiment where the compositions are oil-in-water emulsions, at least one complex according to the invention is in the continuous aqueous phase.

According to one embodiment, the compositions according to the present invention further comprise at least one active agent. In one embodiment, the at least one active agent is selected from the group comprising or consisting of antiseptics such as boric acid, cerium nitrate, povidone-iodine or chlorhexidine, antibiotics as previously discussed, anti-inflammatories such as corticosteroids and non-steroidal anti-inflammatories, and soothing agents such as bisabolol, allantoin, biotin, galacturonic acid, azulene or vitamin E.

In one embodiment, the active agent is povidone-iodine. In one embodiment, the active agent is chlorhexidine.

In one embodiment, the active agent does not interact with the complex of the invention comprised in the composition.

In one embodiment where the composition is an oil-in-water emulsion, the complex of the invention is in the continuous aqueous phase and the at least one active agent is in the lipid (dispersed) phase.

The present invention further relates to a pharmaceutical composition, comprising a composition according to the invention in association with at least one pharmaceutically acceptable excipient.

Suitable pharmaceutically acceptable excipients are well-known from the skilled person in the art. Examples of suitable excipients include, but are not limited to:
- carriers such as water, isopropanol, benzyl alcohol, and propylene glycol;
- stiffening agents;
- rheology modifiers or thickeners such as carbomers such as, for example, Carbopol®, and polyoxyethylene tallow amines;
- surfactants such as anionic, cationic, amphoteric, and non-ionic surfactants, such as, for example, sodium lauryl sulfate, cetostearyl alcohol, cetyl alcohol, magnesium lauryl sulfate, or a combination thereof;
- preservatives such as methyl hydroxybenzoate, hydroxybenzoate, butylparaben, ethylparaben, methylparaben, propyl paraben potassium, propyl paraben sodium; parahydroxybenzoate esters; sorbic acid; potassium sorbate; benzoic acid; parabens; chlorobutanol; phenol; thimerosal; sodium benzoate and benzyl alcohol or a combination thereof;
- humectants,
- buffering agents such as sodium hydroxide, citric acid and potassium hydroxide, potassium phosphate or a combination thereof,
- moisturizing agents and stabilizers.

The pharmaceutical composition according to the invention can be further processed and formulated into a pharmaceutical form.

In one embodiment, the pharmaceutical composition according to the invention is formulated in the pharmaceutical form of:
- aqueous solutions,
- sprays, gels, preferably hydrogels,
- liquid soap formulations, or
- oil-in-water emulsions.

The present invention is also directed to a medicament comprising a complex, a composition or a pharmaceutical composition as previously described.

The invention is equally directed to the use of:
- a complex,
- a composition, or
- a pharmaceutical composition,
according to the invention, for the manufacture of a medicament.

A major advantageous aspect of the present invention consists in the enhancement of the intrinsic aqueous solubility of metals and metal-organic therapeutic agent associations.

In one embodiment, the aqueous solubility of the cyclodextrin-complexed metal or of the cyclodextrin-complexed metal-organic therapeutic agent is multiplied by a factor at least 1.5, at least 2, at least 3, at least 5, at least 7, at least 10, at least 15, at least 20, at least 50, at least 75, at least 100, at least 125, at least 150, at least 200, at least 300, at least 500, at least 1,000, at least 5,000, at least 10,000, at least 50,000 as compared to the intrinsic aqueous solubility of the uncomplexed metal or uncomplexed metal-organic therapeutic agent.

In one embodiment, the composition, pharmaceutical composition or medicament according to the present invention comprises or consist of a metal-cyclodextrin complex or a metal-organic therapeutic agent-cyclodextrin complex as described hereinabove, in an amount ranging from about 0.05 g/L to about 200 g/L, from about 0.05 g/L to about 100 g/L, from about 0.05 g/L to about 50 g/L, from about 0.1 g/L to about 40 g/L, from about 0.2 g/L to about 40 g/L, from about 0.5 g/L to about 40 g/L, from about 0.5 g/L to about 40 g/L, from about 1 g/L to about 40 g/L, from about 1 g/L to about 30 g/L, from about 1 g/L to about 20 g/L, from about 1 g/L to about 10 g/L.

In one embodiment, the composition, pharmaceutical composition or medicament according to the present invention comprises or consists of a metal-cyclodextrin complex or a metal-organic therapeutic agent-cyclodextrin complex as described hereinabove, in an amount ranging from about 0.05 g/L to about 100 g/L, from about 0.05 g/L to about 50 g/L, from about 0.1 g/L to about 40 g/L, from about 0.2 g/L to about 40 g/L, from about 0.5 g/L to about 40 g/L, from about 0.5 g/L to about 40 g/L, from about 1 g/L to about 40 g/L, from about 1 g/L to about 30 g/L, from about 1 g/L to about 20 g/L, from about 1 g/L to about 10 g/L.

In one embodiment, the composition, pharmaceutical composition or medicament according to the present invention comprises or consists of a metal-cyclodextrin complex or a metal-organic therapeutic agent-cyclodextrin complex as described hereinabove, in an amount ranging from about 1 g/L to about 40 g/L, from about 1 g/L to about 30 g/L, from about 1 g/L to about 20 g/L, from about 1 g/L to about 10 g/L.

In one embodiment, the composition, pharmaceutical composition or medicament according to the present invention comprises or consists of a metal-cyclodextrin complex or a metal-organic therapeutic agent-cyclodextrin complex as described hereinabove, in an amount of about 0.1 g/L, about 0.2 g/L, about 0.3 g/L, about 0.4 g/L, about 0.5 g/L, about 0.6 g/L, about 0.7 g/L, about 0.8 g/L, about 0.9 g/L, about 1.0 g/L, about 1.1 g/L, about 1.2 g/L, about 1.3 g/L, about 1.4 g/L, about 1.5 g/L, about 1.6 g/L, about 1.7 g/L, about 1.8 g/L, about 1.9 g/L, about 2.0 g/L, about 3.0 g/L, about 4.0 g/L, about 5.0 g/L, about 6.0 g/L, about 7.0 g/L, about 8.0 g/L, about 9.0 g/L, about 10.0 g/L.

The present invention further relates to a complex, a composition, a pharmaceutical composition or a medicament according to the present invention, for use as a drug.

The invention further relates to a complex, a composition, a pharmaceutical composition or a medicament according to the present invention, for use in the prevention and/or the treatment of a skin infection. The invention further relates to a complex, a composition, a pharmaceutical composition or a medicament according to the present invention, for preventing and/or treating an infection. In one embodiment, the infection is a systemic infection. In one embodiment, the infection is an external infection comprising or consisting of skin infections, mucosal infections, nasal, ophthalmic and auricular infections. In one embodiment, the infection is a skin infection. In one embodiment, the infection is an ophthalmic infection such as bacterial ophthalmic infection or a keratomycosis. In one embodiment, the infection is a bacterial or a fungal auricular infection.

Skin infections include, but are not limited to, bacterial, fungal, viral and parasitic skin infections.

In one embodiment, skin infections include, but are not limited to, gram-positive bacterial external infections, gram-negative bacterial external infections, fungal external infections, viral external infections and parasitic external infections.

In one embodiment, external infections include, but are not limited to, *Staphylococcus aureus* infections, methicillin-resistant *S. aureus* infections, *Staphylococcus epidermidis* infections coagulase-negative *staphylococci* infections, *Enterococcus* spp. infections, vancomycin-resistant *enterococci* infections, *Streptococcus* spp. infections, *Providencia* spp. infections, *Herellea* spp. infections, *Seratia* spp. infections, *Mima* spp. infections, *Citrobacter* spp. infections, *Corynobacterium* spp. infections, *Clostridium* spp. infections, *Pseudomonas aeruginosa* infections, *Escherichia coli* infections, *Klebsiella pneumoniae* infections, *Serratia marcescens* infections, *Enterobacter* spp. infections, *Proteus* spp. infections, *Acinetobacter* spp. infections, *Bacteroides* spp. infections, *Candida* spp. infections, *Cylindrocarpon* spp. infections, *Pseudallescheria* spp. infections, *Culveria* spp. infections, *Curvularia* spp. infections, *Cladosporium* spp. infections, *Penicillium* spp. infections, *Scopulariopsis* spp. infections, *Chrysosporium* spp. infections, *Cephalosporium* spp. infections, *Aspergillus* spp. infections, *Fusarium* spp. infections, *Alternaria* spp. infections, *Rhizopus* spp. infections, *Mucor* spp. infections, Herpes simplex virus infections, Cytomegalovirus infections, Varicella-zoster virus infections, *Balamuthia* infections.

Bacterial external infections include, but are not limited to, impetigo, erysipelas, cellulitis, leprosy, necrotizing fasciitis, ecthyma gangrenosum and myonecrosis.

Fungal external infections include, but are not limited to, athlete's foot, jock itch, ringworm (caused by dermatophytes), yeast infections (such as candidiasis, sporotrichosis) and mycoses.

Viral external infections include, but are not limited to, molluscum contagiosum, shingles (herpes zoster) and chickenpox (varicella).

Parasitic external infections include, but are not limited to, scabies, lice, cutaneous larva migrans, leishmaniasis, tungiasis, myiasis, ticks, creeping eruption, amoebiasis and amebiasis cutis.

In one embodiment, the complex, composition, pharmaceutical composition or medicament according to the present invention are for use in the prevention and/or the treatment of secondary bacterial infections in viral and/or parasitic infections. In one embodiment, the complex, composition, pharmaceutical composition or medicament according to the present invention are for use in the prevention and/or the treatment of secondary bacterial infections in Herpes-simplex (HSV-1 and/or HSV-2) infections.

In one embodiment, the complex, composition, pharmaceutical composition or medicament according to the present invention are for use in the prevention and/or the treatment of a burnt-skin associated infection. Thermal destruction of the skin barrier and concomitant depression of local and systemic host cellular and humoral immune responses are pivotal factors contributing to infectious complications in subjects with severe burns. Burnt-skin infection are well-know from the one skilled in the art. Reference can be made, *e.g*., to Church et al., 2006. Clin Microbiol Rev. 19(2):403-434.

The invention further relates to a complex, a composition, a pharmaceutical composition or a medicament according to the present invention, for use in the prevention and/or treatment of infections associated to skin burns. The invention further relates to a complex, a composition, a pharmaceutical composition or a medicament according to the present invention, for preventing and/or treating skin burns.

Skin burns can be classified according to diagnosis, treatment and prognosis parameters.

In one embodiment, skin burns are first-degree, superficial second-degree, deep second-degree and/or third-degree skin burns.

First-degree skin burns are also termed superficial burns, and affect outer layers of epidermis. They are characterized by an erythema of red color, deep pain, local heat, contact air sensitivity and spontaneous healing in three to four days. First-degree skin burns can produce skin hyper-pigmentation.

Superficial second-degree skin burns partially or completely injure epidermis, but epidermis annex or indentation remain intact. They are characterized by deep pain, erythema, phlycten, fast capillary filling, soft yet skin. Recovery from superficial second-degree skin burns occurs in around 9 days.

Deep second-degree skin burns completely affect and destruct the epidermis, including germinative stratum and part of dermis. They are characterized by phlyctens, pale rose tone, moderate pain (due to nervous destruction), hard and cardboard-like skin, slow capillary filling and delay healing beginning in the annexes (hairs and glands). Recovery from deep second-degree skin burns occurs in around 16 days.

Third-degree skin burns totally compromise the skin, with no cell regeneration. They are characterized by white, insensible, cardboard-like, dry skin without edemas and can involve deeper organs different than skin.

In one embodiment, skin burns are selected from the group comprising or consisting of sun burns, biological burns, steam burns, flame burns, scalds burns, direct fire burns, chemicals burns, contact burns, deflagration burns and electric burns.

In one embodiment, skin burn associated infections are selected from the group comprising or consisting of infections associated to sun burns, biological burns, steam burns, flame burns and scalds burns. In one embodiment, skin burn-associated infections are selected from the group comprising or consisting of infections associated to direct fire burns and chemicals burns. In one embodiment, skin burns are selected from the group comprising or consisting of contact burns, deflagration burns and electric burns.

In one embodiment, the complex, the composition, the pharmaceutical composition or the medicament of the invention is to be administered systemically or locally.

In one embodiment, the complex, the composition, the pharmaceutical composition or the medicament of the invention is to be administered topically, orally, buccally, by injection, by spraying, by topical dispersion of a powder, by ophthalmic instillation, by auricular instillation, by percutaneous administration, parenterally, intraperitoneal, by endoscopy, transdermally, transmucosally, nasally, by inhalation spray, rectally, vaginally, intratracheally, and via an implanted reservoir.

In a preferred embodiment, the complex, the composition, the pharmaceutical composition or the medicament of the invention is to be topically administered. Examples of formulations adapted to topical administration include, but are not limited to, sprays, eye drops, ear drops, sticks, lipsticks, creams, lotions, ointments, balms, gels, powders, leave-on washes or cleansers and/or the like. In one embodiment, the formulation is a spray. In one embodiment, the formulation is an external powder. In one embodiment, the formulation is eye drops. In one embodiment, the formulation is eye drops. In one embodiment, the formulation is a cream, preferably a hydrophilic cream. In one embodiment, the formulation is a gel, preferably a hydrogel. In one embodiment, the formulation is a liquid cleanser.

Topical administration characterizes the delivery, administration or application of the complex, the composition, the pharmaceutical composition or the medicament of the invention directly to the site of interest for a localized effect (generally onto one or more exposed or outer surfaces thereof, such as the outermost layer of the epidermis, which is exposed and visually observable), *e.g*., using hands, fingers or a wide variety of applicators (rollup, roll-on or other stick container, tube container, cotton ball, powder puff, Q-tip, pump, brush, mat, cloth and/or the like). The application may be made, *e.g*., by laying, placing, rubbing, sweeping, pouring, spreading and/or massaging into, or onto, the skin, or by any other convenient or suitable method. Preferably, topical administration is effected without any significant absorption of components of the composition into the subject's blood stream (to avoid a systemic effect).

In one embodiment, the complex, the composition, the pharmaceutical composition or the medicament of the invention is a hydrophilic formulation, preferably a gel, a solution or a spray, and is applied onto the skin in order to completely cover the skin zone to be treated, such as, for example, the wounded or burnt skin.

In one embodiment, the complex, the composition or the pharmaceutical composition of the invention is a liquid soap formulation and is applied on the skin during a bath of a subject in need thereof.

In one embodiment, the present invention also relates to a device comprising the complex, the composition, pharmaceutical composition or medicament of the invention.

In one embodiment, the device comprising the complex, composition, pharmaceutical composition or medicament of the invention is a wound dressing, preferably a dermal patch.

Suitable wound dressings or dermal patches are well-known by the skilled person in the art.

In one embodiment, the wound dressing is a gauze impregnated with the complex, composition, pharmaceutical composition or medicament of the invention.

Ideally, the surface of the wound dressing in contact with the skin is a good absorbent for blood and exudate and does not adhere to the wound surface.

In one embodiment, the wound dressing or dermal patch comprises a hydrophile polymer surface comprising the complex, composition, pharmaceutical composition or medicament of the invention.

Examples of suitable hydrophile polymers include, but are not limited to:
- polymeric polyoxyethylene glycol foams;
- polymeric hydrogels comprising or essentially consisting of polyvinyl alcohol, polyvinyl pyrrolidone, polyethylene oxide, sodium alginate, chitosan, dextran, *N-O*-carboxymethyl chitosan, hydroxyethyl starch, glucan, hyaluronic acid, poly-N-acetylglucosamine, silk proteins or gelatin;
- polymeric cross-linked alginate hydrogels comprising or essentially consisting of sodium alginate crosslinked with Ca, Mg, or Zn salt solutions;
- polymeric hydrocolloides comprising or essentially consisting of iodine/modified starches or crosslinked dextran with polyethylene glycol.

A satisfactory wound dressing creates a suitable microclimate for rapid and effective healing. A good wound dressing meets several criteria:
- it prevents dehydration and scab formation,
- it is permeable to oxygen,
- it is sterilizable,
- it protects against secondary infection,
- it supplies mechanical protection to the wound,
- it is non-toxic,
- it is non-allergenic or sensitizing,
- it does not shed loose material into the wound,
- it conforms to anatomical contours such as flat, convex or concave skin surfaces,
- it resists tearing,
- it resists soiling,
- it is not inflammable,
- it has constant properties in a range of temperatures and humidity,
- it has long shelf life,
- it has small bulk,
- it is compatible with medicaments, and
- it is economical.

In one embodiment, the wound dressing or dermal patch according to the present invention meets at least 1, preferably at least 2, 3, 4, 5, 6, 7, 8, 9, 10, 11, 12, 13, 14, 15, 16 or 17 of the above-listed criteria.

In one embodiment, the wound dressing or dermal patch comprises or consists of:
- a hydrophile polymer surface comprising the complex, composition, pharmaceutical composition or medicament of the invention; and
- a hydrophobic backing film.

In one embodiment, the wound dressing or dermal patch comprises or consists of:
- a hydrophile polymer surface comprising the complex, composition, pharmaceutical composition or medicament of the invention in a density ranging from about 0.5 mg/cm² to about 10 mg/cm²; and
- a hydrophobic backing film.

Examples of suitable hydrophobic backing films include, but are not limited to, polyurethane polymers and silicone/polyester polymers.

In another embodiment, the complex, the composition, the pharmaceutical composition or the medicament of the invention is to be injected, preferably systemically injected. Examples of formulations adapted to systemic injections include, but are not limited to, liquid solutions or suspensions, solid forms suitable for solution in, or suspension in, liquid prior to injection.

Examples of systemic injections include, but are not limited to, intravenous, intratumoral, intracranial, intralymphatic, intraperitoneal, intramuscular, subcutaneous, intradermal, intraarticular, intrasynovial, intrasternal, intrathecal, intravesical, intrahepatic, intralesional, infusion techniques and perfusion. In another embodiment, when injected, the composition, the pharmaceutical composition or the medicament of the invention is sterile. Methods for obtaining a sterile pharmaceutical composition include, but are not limited to, GMP synthesis (GMP stands for "Good manufacturing practice").

In another embodiment, the complex, the composition, the pharmaceutical composition or the medicament of the invention is to be orally administered. Examples of formulations adapted to oral administration include, but are not limited to, solid forms, liquid forms and gels. Examples of solid forms adapted to oral administration include, but are not limited to, pill, tablet, capsule, soft gelatine capsule, hard gelatine capsule, caplet, compressed tablet, cachet, wafer, sugar-coated pill, sugar coated tablet, or dispersing/or disintegrating tablet, powder, solid forms suitable for solution in, or suspension in, liquid prior to oral administration and effervescent tablet. Examples of liquid form adapted to oral administration include, but are not limited to, solutions, suspensions, drinkable solutions, elixirs, sealed phial, potion, drench, syrup and liquor.

In one embodiment, the complex, the composition, the pharmaceutical composition, the medicament or the device according to the present invention is to be administered at a dose determined by the skilled artisan and personally adapted to each subject. It will be understood that the total daily usage of the complex, the composition, the pharmaceutical composition, the medicament or the device according to the present invention will be decided by the attending physician within the scope of sound medical judgment. Dosage is adjusted to provide sufficient levels of the complex, the composition, the pharmaceutical composition, the medicament or the device according to the present invention or to maintain the desired effect of reducing signs or symptoms of the targeted disease, disorder or condition, or reducing severity of the disease, disorder or condition. The specific therapeutically effective amount for any particular patient will depend upon a variety of factors including, but not limited to, the disease, disorder or condition being treated; the severity of the disease, disorder or condition; the prognosis of the disease; the specific composition employed; the time and frequency of administration, route of administration, the duration of the treatment; drugs used in combination or coincidental with the complex, the composition, the pharmaceutical composition, the medicament or the device according to the present invention; reaction sensitivities; tolerance/response to therapy; general health of the subject; age, weight, gender and diet of the subject; and like factors well known in the medical arts.

In one embodiment, a therapeutically effective amount of the complex, the composition, the pharmaceutical composition, the medicament or the device according to the present invention is to be administered at least once a day, at least twice a day, at least three times a day.

In a preferred embodiment, the complex, the composition, the pharmaceutical composition, the medicament or the device according to the present invention is to be administered twice a day, preferably once in the morning and once in the evening.

In one embodiment, the complex, the composition, the pharmaceutical composition, the medicament is instilled from at least one time to eight times per day. In one embodiment, the complex, the composition, the pharmaceutical composition, the medicament is instilled from at least two times to eight times per day. In one embodiment, the complex, the composition, the pharmaceutical composition, the medicament is instilled from at least one time to six times per day.

In one embodiment, a therapeutically effective amount of the complex, the composition, the pharmaceutical composition, the medicament or the device according to the present invention is to be administered every two, three, four, five, six days.

In one embodiment, a therapeutically effective amount of the complex, the composition, the pharmaceutical composition, the medicament or the device according to the present invention is to be administered twice a week, every week, every two weeks, every three weeks, once a month.

In one embodiment of the invention, the complex, the composition, the pharmaceutical composition, the medicament or the device according to the present invention is to be administered for a time period of at least one week, preferably at least two weeks, more preferably at least 3, 4, 5, 6 weeks or more.

In one embodiment, the complex, the composition, the pharmaceutical composition, the medicament to be administered or the dosage applied via a device according to the present invention to a subject ranges from about 0.05 mg/day to about 1.5 g/day. In one embodiment, the dosage ranges from about 0.1 mg/day to about 1.0 g/day. In one embodiment, the dosage ranges from about 0.5 mg/day to about 1.0 g/day. In one embodiment, the dosage ranges from about 5 mg/day to about 1.0 g/day. In one embodiment, the dosage ranges from about 10 mg/day to about 1.0 g/day. In one embodiment, the dosage ranges from about 25 mg/day to about 1.0 g/day.

In one embodiment, the dosage ranges from about 50 mg/day to about 1.0 g/day. In one embodiment, the dosage ranges from about 100 mg/day to about 1.0 g/day. In one embodiment, the dosage ranges from about 200 mg/day to about 1.0 g/day. In one embodiment, the dosage ranges from about 300 mg/day to about 1.0 g/day. In one embodiment, the dosage ranges from about 400 mg/day to about 1.0 g/day. In one embodiment, the dosage ranges from about 500 mg/day to about 1.0 g/day. In one embodiment, the dosage is about 300, about 350, about 400, about 450, about 500, about 550, about 600, about 700, about 750, about 800, about 850, about 900, about 950 or about 1000 mg/day.

One skilled in the art can determine the dosage depending on the surface and the sensibility of an infected area or tissue.

In one embodiment of the invention, the daily amount of the complex, the composition, the pharmaceutical composition, the medicament to be administered or the dosage applied via a device according to the present invention to a subject ranges from about 0.001 to 1000 g/m² of infected area. In one embodiment, the dosage ranges from about 0.005 to about 1000 g/m² of infected area. In one embodiment, the dosage ranges from about 0.01 to about 1000 g/m² of infected area. In one embodiment, the dosage ranges from about 0.05 to about 1000 g/m² of infected area. In one embodiment, the dosage ranges from about 0.1 to about 1000 g/m² of infected area. In one embodiment, the dosage ranges from about 1 to about 1000 g/m² of infected area. In one embodiment, the dosage ranges from about 1 to about 1000 g/m² of infected area. In one embodiment, the dosage ranges from about 1 to about 800 g/m² of infected area. In one embodiment, the dosage ranges from about 5 to about 900 g/m² of infected area. In one embodiment, the dosage ranges from about 10 to about 900 g/m² of infected area. In one embodiment, the dosage ranges from about 10 to about 800 g/m² of infected area. In one embodiment, the dosage ranges from about 5 to about 700 g/m² of infected area. In one embodiment, the dosage ranges from about 10 to about 600 g/m² of infected area. In one embodiment, the dosage ranges from about 50 to about 500 g/m² of infected area.

In one embodiment, the complex, the composition, the pharmaceutical composition, the medicament or the device according to the present invention is to be administered at a dose ranging from about 0.1 mg to about 1500 mg, from about 0.2 mg to about 1500 mg , from about 0.5 mg to about 1500 mg, from about 1 mg to about 1500 mg, from about 5 mg to about 1500 mg, from about 1 mg to about 1000 mg, from about 5 mg to about 1000 mg, from about 0.1 mg to about 900 mg, from about 0.1 mg to about 800 mg, from about 0.1 mg to about 700 mg, from about 1 mg to about 800 mg, from about 5 mg to about 700 mg, from about 10 mg to about 600 mg, from about 20 mg to about 700 mg, from about 50 mg to about 1500 mg, from about 50 mg to about 1000 mg, from about 50 mg to about 900 mg or from about 100 mg to about 1000 mg.

In one embodiment, the subject to whom the complex, the composition, the pharmaceutical composition, the medicament or the device according to the present invention is to be administered is a mammal. In one embodiment, the mammal is a human.

In one embodiment, the mammal is an animal. In one embodiment, the animal is selected from a group comprising or consisting of farm and pet animals. In one embodiment, the mammal is selected from a group comprising or consisting of cats, dogs, horses, donkeys and ruminants such as cattle, goats and sheep. In one embodiment, the mammal is a dog. In one embodiment, the mammal is a horse.

In one embodiment, the subject to whom the complex, the composition, the pharmaceutical composition, the medicament or the device according to the present invention is to be administered is at risk for developing or is affected by, preferably is diagnosed with, a skin infection, preferably a skin infection related to a skin burn. In one embodiment, the subject is diagnosed with skin infection, preferably with a skin infection related to a skin burn. In one embodiment, the subject is at high risk of developing a skin infection, preferably a skin infection related to a skin burn. In one embodiment, the subject presents skin lesions. In one embodiment, the subject presents skin lesions related to a skin burn.

In one embodiment, the subject to whom the complex, the composition, the pharmaceutical composition, the medicament or the device according to the present invention is to be administered is at risk for developing or is affected by, preferably is diagnosed with, on ophthalmic infection.

In one embodiment, the subject to whom the complex, the composition, the pharmaceutical composition, the medicament or the device according to the present invention is to be administered is at risk for developing or is affected by, preferably is diagnosed with, on auricular infection.

The invention further relates to a method of treatment and/or prevention of a subject in need thereof. In one embodiment, the method comprises administering a pharmaceutically effective amount of a complex, a composition, a pharmaceutical composition, a medicament or a device according to the present invention to the subject.

The invention further relates to a method for treating and/or preventing skin infections in a subject in need thereof, said method comprising administering a pharmaceutically effective amount of a complex, a composition, a pharmaceutical composition, a medicament or a device according to the present invention to the subject.

The invention further relates to a method for treating and/or preventing bacterial and/or fungal skin infections in a subject in need thereof, said method comprising administering a pharmaceutically effective amount of a complex, a composition, a pharmaceutical composition, a medicament or a device according to the present invention to the subject.

The invention further relates to a method for treating and/or preventing skin burn associated infections in a subject in need thereof, said method comprising administering a pharmaceutically effective amount of a complex, a composition, a pharmaceutical composition, a medicament or a device according to the present invention to the subject.

The invention further relates to a method for treating and/or preventing ophthalmic infections in a subject in need thereof, said method comprising administering a pharmaceutically effective amount of a complex, a composition, a pharmaceutical composition, a medicament or a device according to the present invention to the subject.

The invention further relates to a method for treating and/or preventing auricular, preferably external ear, infections in a subject in need thereof, said method comprising administering a pharmaceutically effective amount of a complex, a composition, a pharmaceutical composition, a medicament or a device according to the present invention to the subject.

The present invention further relates to the use of a complex, a composition, a pharmaceutical composition, a medicament or a device according to the present invention, for the manufacture of a medicament.

The invention further relates to the use of a complex, a composition, a pharmaceutical composition, a medicament or a device according to the present invention, for the manufacture of a medicament for the prevention and/or the treatment of a skin infection.

The invention further relates to the use of a complex, a composition, a pharmaceutical composition, a medicament or a device according to the present invention, for the manufacture of a medicament for the prevention and/or the treatment of bacterial and/or fungal skin infections.

The invention further relates to the use of a complex, a composition, a pharmaceutical composition, a medicament or a device according to the present invention, for the manufacture of a medicament for the prevention and/or treatment of skin burn associated infections.

The invention further relates to the use of a complex, a composition, a pharmaceutical composition, a medicament or a device according to the present invention, for the manufacture of a medicament for the prevention and/or treatment of ophthalmic infections.

The invention further relates to the use of a complex, a composition, a pharmaceutical composition, a medicament or a device according to the present invention, for the manufacture of a medicament for the prevention and/or treatment of auricular, preferably external ear, infections.

The present invention further relates to the use of a complex, a composition, a pharmaceutical composition, a medicament or a device according to the present invention as a medicament.

The invention further relates to the use of a complex, a composition, a pharmaceutical composition, a medicament or a device according to the present invention, for preventing and/or treating a skin infection.

The invention further relates to the use of a complex, a composition, a pharmaceutical composition, a medicament or a device according to the present invention, for preventing and/or treating bacterial and/or fungal skin infections.

The invention further relates to the use of a complex, a composition, a pharmaceutical composition, a medicament or a device according to the present invention, for preventing and/or treating skin burn associated skin infections.

The invention further relates to the use of a complex, a composition, a pharmaceutical composition, a medicament or a device according to the present invention, for preventing and/or treating ophthalmic infections.

The invention further relates to the use of a complex, a composition, a pharmaceutical composition, a medicament or a device according to the present invention, for preventing and/or treating auricular, preferably external ear, infections.

The invention further relates to the use of a complex, a composition, a pharmaceutical composition, a medicament or a device according to the present invention, for preventing and/or treating secondary bacterial infections of viral infections.

The invention further relates to a method for improving the aqueous solubility of a metal selected from the group comprising or consisting of Ag, Zn, Cu, Pt, Au, Ru, As, Sb, Bi, Ti, V, Ni, Hg, Pb, Co, oxides, hydroxides and salts thereof, wherein the method comprises providing a reaction mixture comprising said metal, an aqueous medium and a cyclodextrin of formula (I): 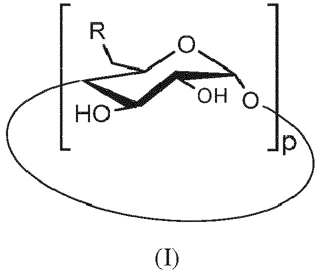 wherein:
p is 5, 6, 7 or 8;
R is -OH or -X-NH-Z-NHR₁
X is a single bond or R₂;
Z is selected -C=O and -C=S;
R₁ is selected from H or optionally substituted short chain alkyls; preferably
R₁ is selected from methyl, ethyl and propyl; and
R₂ is selected optionally substituted short chain alkyls; preferably R₁ is selected from methyl, ethyl and propyl;
with the proviso that at least one beta-cyclodextrin monomer has R: -X-NH-Z-NHR₁.

In one embodiment, the method is for improving the aqueous solubility of a metal selected from the group comprising or consisting of Ag, Zn, Cu, Pt, Au, Ru, As, Sb, Bi, oxides, hydroxides and salts thereof.

In one embodiment, the method is for improving the aqueous solubility of a metal selected from the group comprising or consisting of Ag, Zn, Cu, Pt, Au, Ru, As, oxides, hydroxides and salts thereof.

In one embodiment, the method is for improving the aqueous solubility of a metal selected from the group comprising or consisting of Ag, Zn, Cu, Pt, Au, Ru, As, oxides, hydroxides and salts thereof.

In one embodiment, the method is for improving the aqueous solubility of a metal selected from the group comprising or consisting of Ag, Zn, Cu, Pt, Au, oxides, hydroxides and salts thereof.

In one embodiment, the reaction mixture further comprises at least one organic therapeutic agent as previously described.

In one embodiment, the metal is Ag, oxides, hydroxides or salts thereof; and the least one organic therapeutic agent is selected from antibiotic, anti-fungal, antiviral and antiparasitic agents, salts thereof and combinations thereof; preferably the organic therapeutic agent is sulfamide, even more preferably the organic therapeutic agent is sulfadiazine.

In one embodiment, the metal is Ag and the least one organic therapeutic agent is selected from antibiotic, anti-fungal, antiviral and antiparasitic agents, salts thereof and combinations thereof; preferably the organic therapeutic agent is sulfadiazine.

In one embodiment, the metal is Zn and the least one organic therapeutic agent is selected from antibiotic, anti-fungal, antiviral and antiparasitic agents; salts thereof and combinations thereof; preferably the organic therapeutic agent is pyrithione.

In one embodiment:
(i) the metal is selected from the group comprising or consisting of Ag, Zn, Cu, Pt, Au, Ru, As, oxides, hydroxides and salts thereof;
(ii) the least one organic therapeutic agent is selected from antibiotic, anti-fungal, antiviral and antiparasitic agents, salts thereof and combinations thereof; and
(iii) the cyclodextrin of formula (I) is an alpha or beta-cyclodextrin, wherein:
   p is 6 or 7;
   R is -OH or -X-NH-Z-NHR₁;
   X is a single bond or R₂;
   Z is -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁.

In one embodiment:
(i) the metal is Zn or Cu oxides, hydroxides and salts thereof;
(ii) the least one organic therapeutic agent is selected from antibiotic, anti-fungal, antiviral and antiparasitic agents, salts thereof and combinations thereof; and
(iii) the cyclodextrin of formula (I) is an alpha or beta-cyclodextrin, wherein:
   p is 6 or 7;
   R is -OH or -X-NH-Z-NHR₁;
   X is a single bond or R₂;
   Z is -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁.

In one embodiment:
(i) the metal is Ag;
(ii) the least one organic therapeutic agent is selected from antibiotic, anti-fungal, antiviral and antiparasitic agents, salts thereof and combinations thereof; preferably the organic therapeutic agent is sulfamide, even more preferably the organic therapeutic agent is sulfadiazine; and
(iii) the cyclodextrin of formula (I) is an alpha or beta-cyclodextrin, wherein:
   p is 6 or 7;
   R is -OH or -X-NH-Z-NHR₁;
   X is a single bond or R₂;
   Z is -C=S;
   R₁ is selected from H and optionally substituted short chain alkyls; preferably
   R₁ is selected from methyl, ethyl propyl and butyl;
   R₂ is selected from optionally substituted short chain alkyls; preferably R₂ is selected from methyl, ethyl and propyl;
   with the proviso that at least one R = -X-NH-Z-NHR₁.

In one embodiment:
(i) the metal is Ag;
(ii) the least one organic therapeutic agent is selected from antibiotic, anti-fungal, antiviral and antiparasitic agents, salts thereof and combinations thereof; preferably the organic therapeutic agent is sulfamide, even more preferably the organic therapeutic agent is sulfadiazine; and
(iii) the cyclodextrin of formula (I) is a beta-cyclodextrin, wherein:
   p is 7;
   R is -OH or -X-NH-Z-NHR₁;
   X is a single bond;
   Z is -C=S;
   R₁ is methyl;
   with the proviso that at least one R = -X-NH-Z-NHR₁.

### BRIEF DESCRIPTION OF THE DRAWINGS

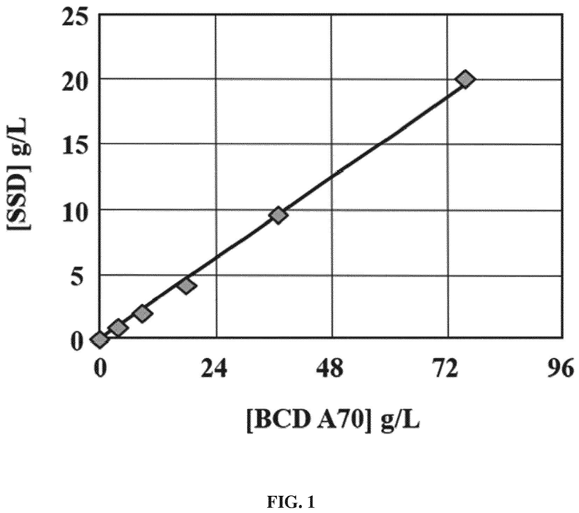

**Figure 1** is a solubility diagram of silver sulfadiazine with the beta-cyclodextrin A70 of formula (I) according to the present invention. The y-axis represents the concentration of silver sulfadiazine in g/L and the x-axis represents the concentration of BCD A70 in g/L.

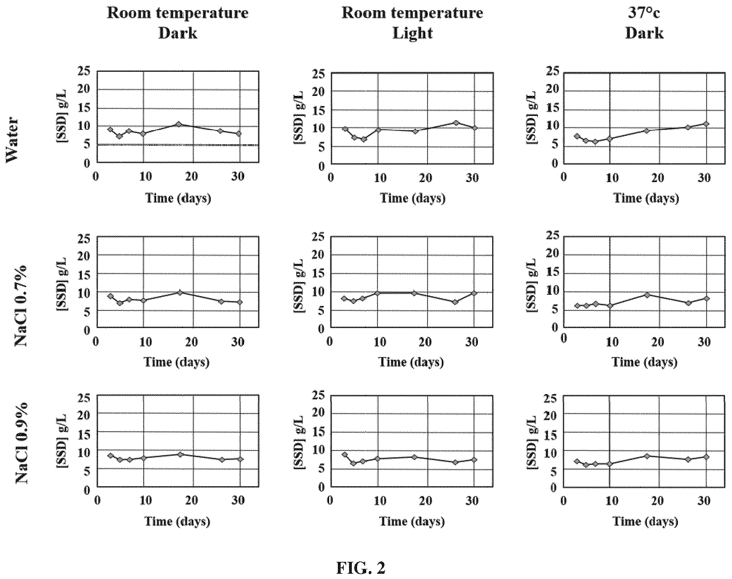

**Figure 2** is a set of nine graphs, showing the stability of silver sulfadiazine when complexed to BCD A70. The y-axis represents the concentration of silver sulfadiazine in g/L and the x-axis represents the time in days. The three graphs of the left column are stability assays carried out at room temperature, in the dark; the middle column shows stability at room temperature, in the light; and the right column shows the stability results at 37°C, in the dark. The three graphs of the upper line show the stability of a 70-SD formulation in water; the middle line, that of a 70-SD formulation in 0.7% NaCl; and on the lower line, the stability of a 70-SD formulation in 0.9% NaCl.

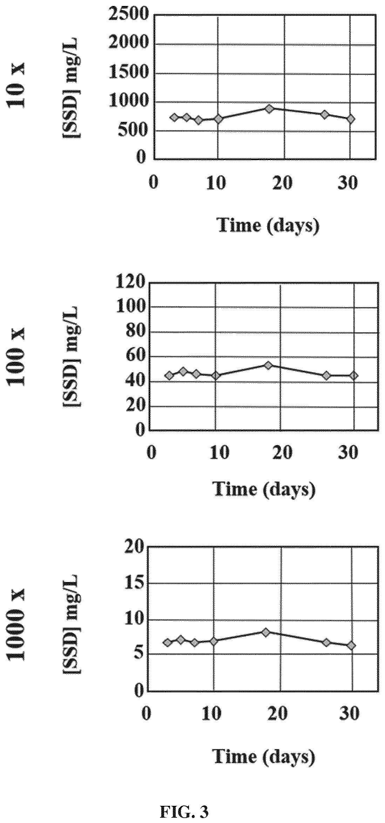

**Figure 3** is a set of three graphs, showing the stability of silver sulfadiazine when complexed to BCD A70. The y-axis represents the concentration of silver sulfadiazine in mg/L and the x-axis represents the time in days. The upper graph shows the stability of a 70-SD formulation diluted 10x; the middle graph shows that of a 70-SD formulation diluted 100x; and the lower graph shows the stability of a 70-SD formulation diluted 1000x.

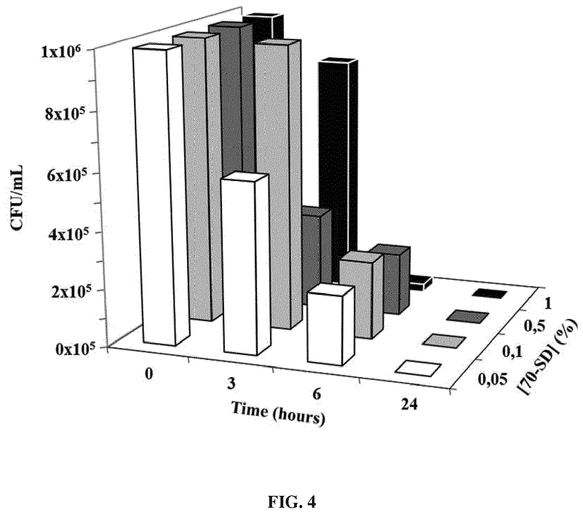

**Figure 4** is a diagram showing the bactericide effect of the 70-SD complex against *Staphylococcus aureus.* The 70-SD complex is tested in formulations at concentrations of 0.05, 0.1, 0.5 and 1% w/w. The y-axis represents the bactericide effect (in CFU/mL), the x-axis represents time of incubation in hours and the z-axis represents the concentration of 70-SD complex tested.

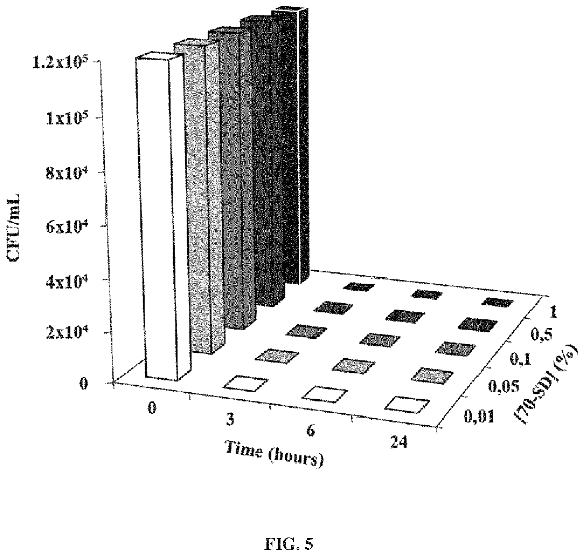

**Figure 5** is a diagram of the bactericide effect of the 70-SD complex against *Pseudomonas aeruginosa.* The 70-SD complex is tested in formulations at concentrations of 0.01, 0.05, 0.1, 0.5 and 1% w/w. The y-axis represents the bactericide effect (in CFU/mL), the x-axis represents time of incubation in hours and the z-axis represents the concentration of 70-SD complex tested.

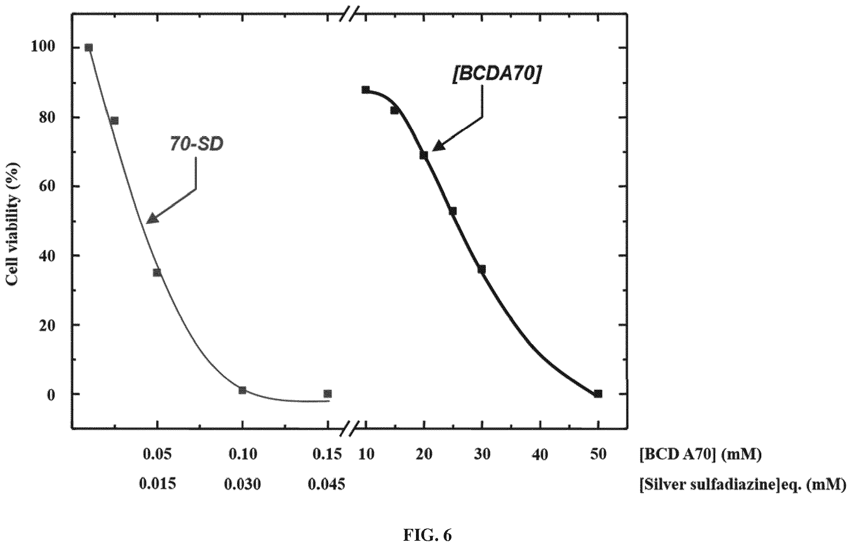

**Figure 6** is a graph showing the viability of SIRC cell line past the treatment with the cyclodextrin carrier alone (BCD A70) and with the 70-SD complex. The y-axis represents the cell viability percentage. The x-axis represents the concentration of BCD A70 in mM (alone or within the 70-SD complex) and the equivalent concentration of silver sulfadiazine within the 70-SD complex in mM.

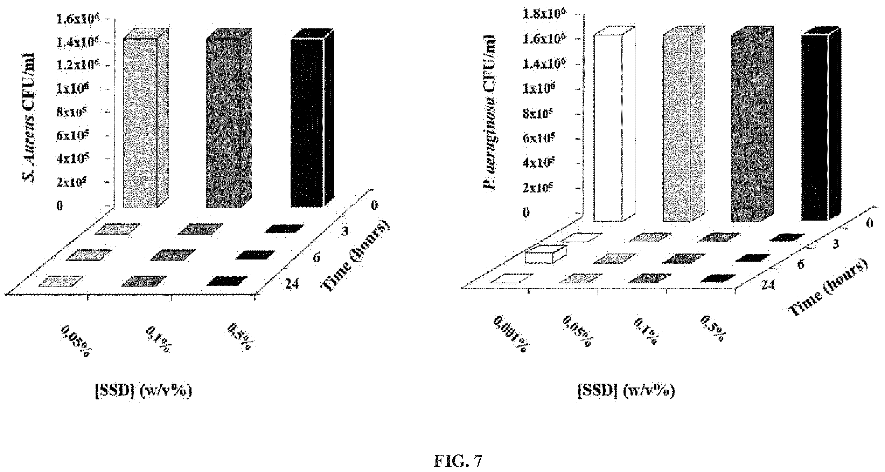

**Figure 7** is a diagram showing the bactericide effect of Flammazine® ointment aqueous dispersions against *Staphylococcus aureus* (left) and *Pseudomonas aeruginosa* (right).

The Flammazine® ointment aqueous dispersions are tested in silver sulfadiazine equivalent concentrations of 0.001, 0.05, 0.1, 0.5 and 1% w/v. The y-axis represents the bactericide effect (in CFU/mL), the z-axis represents time of incubation in hours and the x-axis represents the concentration of the equivalent silver sulfadiazine concentrations tested.

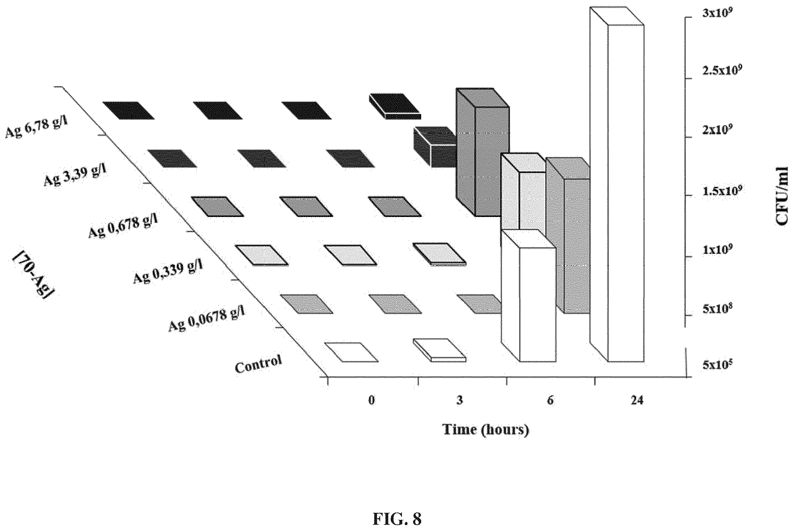

**Figure 8** is a diagram showing the antimicrobial effect of 70-Ag against *Staphylococcus aureus.* 70-Ag is tested in silver equivalent concentrations of 0.0678, 0.339, 0.678, 3.39 and 6.78 g/L. The y-axis represents the bactericide effect (in CFU/mL), the x-axis represents time of incubation in hours and the z-axis represents the concentration of the equivalent silver concentrations tested.

### EXAMPLES

The present invention is further illustrated by the following examples.

### Abbreviations

"70-SD" refers to the complex of beta-cyclodextrin BCD A70 with silver sulfadiazine.

"70-Ag" refers to the complex of beta-cyclodextrin BCD A70 with silver ions.

"BCD A70" corresponds to the beta-cyclodextrin of formula (I) 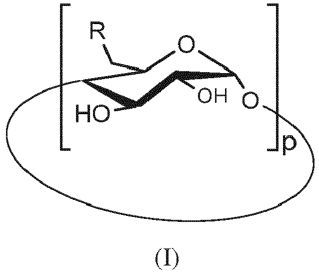 wherein:
p is 7;
R is -OH or -NH-Z-NHR₁;
Z is -C=S; and Ri is methyl;
with the proviso that one beta-cyclodextrin monomer has R = -NH-Z-NHR1.

"BCD A56" corresponds to 6-monopropanediamino-β-cyclodextrin.

"CFU" corresponds to colony-forming unit, *i.e*., a unit used to estimate the number of viable microorganism, preferably of viable bacteria, in a sample.

"SSD" corresponds to silver sulfadiazine.

### Example 1: SSD solubility enhancement

SSD was mixed with BCD A70 and aqueous solubility of the complex was assessed.

**Figure 1** shows that addition of BCD A70 remarkably increases SSD solubility in a proportional manner. Indeed, the BCD A70-SSD interaction is such that the complexation constant is estimated at 160 000 M⁻¹. The weight ratio of SSD:BCD A70 is calculated around 1:3.3-3.5, corresponding to a molar ratio close to 1:1.

Optimization of the complexation conditions leads to a maximal solubility of SSD around 59 g/L (*i.e*., 165 mM), with an aqueous solubility 53,000 times higher than its intrinsic solubility.

In conclusion, from a nearly water-insoluble SSD preparation, the Applicant has shown that the complex of the invention achieves significantly greater aqueous concentrations than the non-complexed SSD or SSD-cyclodextrin complexes of the art. The high concentration of SSD in the complex of the invention makes it possible to lyophilize and store an easily and rapidly dissoluble SSD preparation.

### Example 2: silver:sulfadiazine molar ratio

In order to determine the silver:sulfadiazine ratio in the complexes of the invention, quantitative solubilizing in trifluoroacetic acid (TFA) was performed. Briefly, SSD was dissolved in 100% TFA and analyzed by Inductively Coupled Plasma/Optical Emission Spectrometry (ICP/OES) on an Optima 2000 DV apparatus (Perkin Elmer).

Results are given in **Table 1.**

In non-complexed SSD, the molar ratio between silver and sulfadiazine was estimated to 1:1.

The Applicant further tested SSD in complex with cyclodextrins, to confirm maintenance of this ratio. Two cyclodextrin complexes were tested:
- the SSD-BCD A70 complex (70-SD), object of the present invention, and
- a SSD-BCD A56 complex.

As shown in **Table 1**, the stoichiometric silver:sulfadiazine molar ratio in the BCD A56 complex is not maintained, with a measured 1:222 molar ratio. On the contrary, the 70-SD maintains a near equimolar ratio of 1:1.3, close to the ratio measured in uncomplexed SSD.

**Table 1: [Ag] / [sulfadiazine] mass and molar ratios in uncomplexed SSD, SSD-BCD A56 complex and SSD-BCD A70 complex (70-SD) of the present invention.**

| | **[Ag] / [sulfadiazine]** | |
|---|---|---|
| | **Molar ratio** | **Mass ratio** |
| **Uncomplexed SSD** | 1:1 | 1:2.3 |
| **SSD-BCD A56 complex** | 1:222 | 1:518 |
| **70-SD** | 1:1.3 | 1:3.1 |

Advantageously, the complex of the present invention achieves significantly greater aqueous concentrations than the non-complexed SSD or SSD-cyclodextrin complexes of the art, while maintaining substantially equimolar ratios between the metal and the organic therapeutic agent. Such an effect is of particular interest since the synergistic effect between the metal and the organic therapeutic agent provides SSD its effectiveness.

### Example 3: solutions stability

The stability of three 70-SD aqueous solutions (in water, in NaCl 0.7% and in NaCl 0.9%) was assessed in various conditions:
- at room temperature in the dark, over one month,
- at room temperature in the light, over one month,
- at 37°C in the dark, for one month.

A standard silver sulfadiazine solution was prepared by solubilizing 40 g of silver sulfadiazine in 100% TFA and diluted to 1/1000 in pure water, yielding a 20 mg/L standard silver sulfadiazine solution. The standard and sample solutions were analyzed by HPLC using a Chromolith™ Performance (RP-18e 4,6 mm x 100 mm) column using a UV detection at 254 nm. The eluent consisted of solvents A (H₂O comprising 0.1% w/w TFA) and B (Methanol) and was pumped at a flow rate of 1 mL/min. The used eluent gradient was as follows :
- 0-3.5 min, 100% solvent A -0% solvent B
- 3.5-7 min, gradually raising solvent B to 100%
- 7 min-12, 100% solvent B
- 12-15 min, 100% solvent A.

The results are presented in **Figure 2****.**

The Applicant surprisingly demonstrated that not only are cyclodextrin complexes advantageous in terms of solubility enhancement and preservation of metal/organic therapeutic agent molar ratio within the complex, but also that the complexes of the present invention are particularly stable, whether in the dark or the light, at room temperature or at 37°C, in water or in saline solution up to 0.9% NaCl.

The Applicant further assessed the stability of the 70-SD aqueous solution upon dilution. Results are presented in **Figure 3****.**

Similarly, the Applicant has shown that the cyclodextrin complexes of the present invention are also stable regardless of any dilution effect.

### Example 4: antimicrobial effect

The antimicrobial effect of the 70-SD complex formulation was tested on several bacterial strains. To demonstrate efficacy of the formulation, it was necessary to test it in complex media favoring microbial development. Namely, brain heart infusion (BHI) medium was used. Bacteria were seeded at high concentration (10⁶ bacterial cells/mL), incubated at 37°C and counted as a function of time.

Results obtained with a *Staphylococcus aureus* DSMZ 799 strain are presented in **Figure 4****.** These results show a slow release of the organic therapeutic agent with a maximal bactericide effect seen after 24 hours, characterized by the disappearance of the target bacteria.

Tests were carried out with other bacterial strains, showing a faster bactericide effect, as seen with a *Pseudomonas aeruginosa* DSMZ 1128 strain. These results are presented in **Figure 5****.** Among the other bacterial strains tested, a rapid bactericide effect was also observed against *Enterococcus faecalis*, *Klebsiella pneumoniae* and *Escherichia coli* strains past the treatment with 70-SD. The results are presented in **Table 2.**

**Table 2: Enterococcus faecalis, Klebsiella pneumoniae and Escherichia coli viability inhibition by the 70-SD complex of the present invention in a 0.1% w/w concentration after 24 hours. The bacterial viability is expressed in CFU/mL.**

| **Hours** | ***Klebsiella pneumoniae* Strain 0502083** | | ***Pseudomonas aeruginosa* Strain 9107054** | |
|---|---|---|---|---|
| | Control | [70-SD] 0.1% | Control | [70-SD] 0.1% |
| **0** | 8.50 x10³ | 8.50 x10³ | 9.50 x10³ | 9.50 x10³ |
| **24** | 4.6 x10⁹ | 0 | 9.18x10⁹ | 0 |
| | | | | |

| **Hours** | ***Enterococcus faecalis* Strain 900117** | | ***Escherichia coli* Strain 8144107** | |
|---|---|---|---|---|
| | Control | [70-SD] 0,1% | Control | [70-SD] 0.1% |
| **0** | 1.14 x10⁴ | 1.14 x10⁴ | 1.6 x10⁴ | 1.6 x10⁴ |
| **24** | 4.03 x10⁹ | 0 | 5.52 x10⁹ | 0 |

Finally, a bacteriostatic effect was observed against *Listeria monocytogenes* and *Bacillus subtilis.* These results are presented in **Table 3.**

**Table 3: Listeria monocytogenes and Bacillus subtilis viability inhibition by the 70-SD complex of the present invention in a 0.1% w/w concentration after 24 hours. The bacterial viability is measured in CFU/mL.**

| **Hours** | ***Listeria monocytogenes* Strain 8309028** | | ***Bacillus subtilis* Strain 9802005** | |
|---|---|---|---|---|
| | Control | [70-SD] 0.1% | Control | [70-SD] 0.1% |
| **0** | 1.88 x10⁴ | 1.88 x10⁴ | 8.70 x10³ | 8.70 x10³ |
| **24** | 7.80 x10⁸ | 3.00 x10³ | 5.20 x10⁷ | 3.87 x10⁴ |

### Example 5: absence of toxicity

Toxicity assays were carried out on non-cancerous animal cells (rabbit corneal cell line, SIRC). Cell viability was assessed by ATPlite assay (Perkin Elmer), in presence of 70-SD or BCD A70 alone.

Results are presented in **Figure 6****.** These results show that the cyclodextrin carrier (BCD A70) has no cytotoxic effect at used concentrations, and that the 70-SD complex exhibits the same results as uncomplexed SSD, suggesting that the complexation with the BCD A70 cyclodextrin does not modify the biological properties of the organic therapeutic agent.

### Example 6: Flammazine® aqueous dispersions antimicrobial effect

The antimicrobial effect of Flammazine® was assessed against *Staphylococcus aureus* DSMZ 799 strain and *Pseudomonas aeruginosa* DSMZ 1128 using the same protocol as in Example 2.

Flammazine® ointment (1% w/w or 0.958% w/v silver sulfadiazine) and a 1/10 aqueous dispersion thereof were used to prepare the samples. The tested Flammazine® samples contained 0.01%, 0.05%, 0.1% and 0.5% w/v silver sulfadiazine.

The 0.5% w/v sample was presented high viscosity, hindering its handling and limiting the precision of the volume sampling.

The results are presented in **Figure 7****.**

The 70-SD complex formulations of the invention presented the same antimicrobial effect (kill-rate) as the Flammazine® ointment. Furthermore, the negative influence of the ointment excipients on the bacterial viability should be taken into consideration.

### Example 7: 70-Ag antimicrobial effect

The complex 70-Ag was assessed for its antimicrobial effects. The antimicrobial effect of the 70-Ag complex formulation was tested on *Staphylococcus aureus* DSMZ 799 strain are presented in **Figure 8****.** Five samples of 70-Ag were tested, having an equivalent Ag concentration of 0.067, 0.339, 0.678, 3.39 and 6.78 g/L.

These results show an effective release of silver ions over short time (up to six hours after treatment) intervals.

Similar results were obtained with the 70-Ag antimicrobial assessment against *Pseudomonas aeruginosa* DSMZ 1128 strain.

### Conclusion

Altogether, these data demonstrate the efficacy of the beta-cyclodextrin of formula (I) (BCD A70) to solubilize SSD, while maintaining substantially equimolar ratios between the silver and sulfadiazine within the complex. This is clearly in contrast with what has been observed with other cyclodextrins such as BCD A56. Maintaining the metal:organic therapeutic agent ratio in an equimolar range is of particular importance since the effectiveness of SSD rests on the combined effect of both silver ions and sulfadiazine.

The Applicant has also demonstrated that 70-SD formulations are stable over time, regardless of the brightness, temperature, salt concentration or dilution factor.

A strong antimicrobial effect was shown on several Gram-positive and Gram-negative bacterial strains, with no cytotoxicity for mammalian cells at used dosage. Advantageously, the beta-cyclodextrin of the invention showed no toxic effects for sensitive non-cancerous mammalian corneal cells.

Compared to commercial products comprising silver sulfadiazine, the 70-SD complex comprising the same amount of silver sulfadiazine yielded the same antimicrobial effects without being limited to the dispersion of the treatment in an aqueous medium.

Furthermore, the 70-Ag complex showed an effective short-time interval antimicrobial effect.

There are to date no equivalent of 70-SD on the market. Indeed, SSD formulations currently available make use of detergents such as polysorbates and esters and fatty acids. 70-SD is advantageous in several aspects: with its increased aqueous solubility, SSD in complex with BCD A70 can easily be sprayed on injured tissues and wounds, thereby overcoming additional pain issues encountered when applying creams, balms or ointments. As a prophylaxis, baths used to relieve infections of severe burnt subjects can be supplemented with 70-SD, or fabrics and clothing be sprayed and/or soaked with 70-SD.

Lastly, current lipid SSD formulations cannot be instilled into subjects' eyes or ears, thus limiting the therapeutic potential of an active agent belonging listed as a World Health Organization's essential medicines. The present invention paves the way to the valorization of SSD in ophthalmic and auricular applications, without the concomitant drawbacks of greasy excipients in such sensitive tissues.

## Claims

1. A complex of a metal selected from the group comprising Ag, Zn, Cu, Pt, Au, oxides, hydroxides and salts thereof; with a cyclodextrin of formula (I) 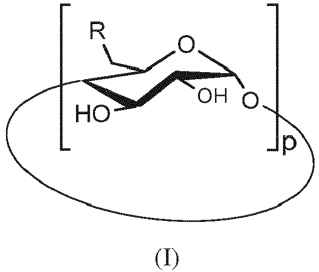 wherein:
p is 6, 7 or 8;
R is -OH or -X-NH-Z-NHR₁;
X is a single bond or R₂;
Z is selected from -C=O and -C=S;
R₁ is selected from H and optionally substituted short chain alkyls; preferably
R₁ is selected from methyl, ethyl propyl and butyl;
R₂ is selected from optionally substituted short chain alkyls; preferably R₂ is selected from methyl, ethyl and propyl;
with the proviso that at least one R = -X-NH-Z-NHR₁.

2. The complex according to claim 1, wherein:
p is 7;
R is -OH or -NH-Z-NHR₁;
Z is -C=S;
R₁ is methyl;
with the proviso that at least one cyclodextrin monomer has R = -NH-Z-NHR₁.

3. The complex according to claim **1** or **2**, further comprising at least one organic therapeutic agent, said organic therapeutic agent being selected from antibiotic, anti-fungal, antiviral and antiparasitic agents; salts thereof and combinations thereof.

4. The complex according to claim **3**, wherein
- the metal is Ag, oxide, hydroxide or salts thereof, and
- the organic therapeutic agent is an antibiotic agent, preferably a sulfamide, even more preferably the organic therapeutic agent is sulfadiazine.

5. The complex according to claim **3** or **4**, wherein:
- the metal is Ag;
- the organic therapeutic agent is a sulfamide, preferably sulfadiazine;
- the cyclodextrin is of formula (I), wherein:
p is 7;
R is -OH or -NH-Z-NHR₁;
Z is -C=S; and Ri is methyl;
with the proviso that at least one beta-cyclodextrin monomer has R = -NH-Z-NHR₁; and
- the molar ratio between the organic therapeutic agent and the metal is ranging from about 200 to about 1, preferably from about 20 to about 2, even more preferably from about 2 to 1.

6. A composition comprising the complex according to any one of claims **1** to **5**.

7. The composition according to claim **6**, wherein:
- the metal is Ag;
- the cyclodextrin is of formula (I), wherein:
p is 7;
R is -OH or -NH-Z-NHR₁;
Z is -C=S;
R₁ is methyl;
with the proviso that at least one beta-cyclodextrin monomer has R = -NH-Z-NHR1; and
- when the complex comprises at least one organic therapeutic agent, said organic therapeutic agent is sulfadiazine; and
- the molar ratio between sulfadiazine and Ag is ranging from about 2 to about 1, preferably from about 1.5 to about 1; and
- the concentration of silver sulfadiazine in the composition is ranging from about 1.0 mg/L to about 40 g/L; preferably ranging from about 1.0 to about 20 g/L.

8. The composition according to claim **6** or **7**, further comprising at least one active agent selected from antiseptics, antibiotics, anti-inflammatories, and soothing agents.

9. A pharmaceutical composition comprising the composition according to any one of claims **6** to **8**, and at least one pharmaceutically acceptable excipient.

10. The pharmaceutical composition according to claim **9**, in a pharmaceutical form selected from aqueous solutions, sprays, gels, hydrogels, liquid soap formulations, eye drops, ear drops and oil-in-water emulsions.

11. The complex according to any one of claims **1** to **5**, the composition according to any one of claims **6** to **8** or the pharmaceutical composition according to claim **9** or **10**, for use as a drug.

12. The complex according to any one of claims **1** to **5**, the composition according to any one of claims **6** to **8** or the pharmaceutical composition according to claim **9** or **10**, for use in the prevention and/or the treatment of skin infections.

13. A device comprising the complex according to any one of claims **1** to **5**, the composition according to any one of claims **6** to **8** or the pharmaceutical composition according to claim **9** or **10**; preferably the device is a wound dressing; even more preferably the device is a dermal patch.

14. A method for improving the aqueous solubility of a metal selected from the group comprising Ag, Zn, Cu, Pt, Au, Ru, As, Sb, Bi, Ti, V, Ni, Hg, Pb, Co, oxides, hydroxides and salts thereof;
wherein the method comprises providing a reaction mixture comprising said metal, an aqueous medium and a cyclodextrin of formula (I): 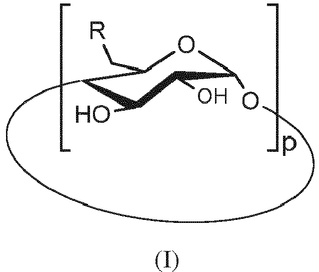 wherein:
p is 5, 6, 7 or 8;
R is -OH or -X-NH-Z-NHR₁
X is a single bond or R₂;
Z is selected -C=O and -C=S;
R₁ is selected from H or optionally substituted short chain alkyls; preferably
R₁ is selected from methyl, ethyl and propyl; and
R₂ is selected optionally substituted short chain alkyls; preferably Ri is selected from methyl, ethyl and propyl;
with the proviso that at least one beta-cyclodextrin monomer has R: -X-NH-Z-NHR₁.

15. The method according to claim **14**, wherein the metal is Ag and the reaction mixture further comprises at least one organic therapeutic agent;
wherein the organic therapeutic agent is selected from antibiotic, anti-fungal, antiviral and antiparasitic agents; salts thereof and combinations thereof; preferably the organic therapeutic agent is sulfamide, even more preferably the organic therapeutic agent is sulfadiazine.
